# EUROPEAN PATENT SPECIFICATION

(11) **EP 0 959 132 B1**
(45) Date of publication and mention of the grant of the patent: **23.08.2006**
(21) Application number: 97943188.9
(22) Date of filing: 13.10.1997
(51) Int. Cl.: C12N 15/57, C12N 9/48, C12Q 1/68, G01N 33/574

(54) **NUCLEIC ACID ENCODING RECEPTOR TYPE PROTEIN KINASE**
NUKLEINSÄURE, DIE FÜR FÜR EINE REZEPTOR-PROTEINKINASE KODIERT.
ACIDE NUCLEIQUE CODANT UNE PROTEINE KINASE DU TYPE RECEPTEUR

(30) Priority: 18.10.1996 JP 29732996
(43) Date of publication of application: 24.11.1999
(73) Proprietor: TAKARA BIO INC., Otsu-shi, Shiga (JP)
(72) Inventor: YOKOTA, Shohei, Kyoto 603 (JP)
(74) Representative: Vossius & Partner
(86) International application number: PCT/JP1997/003667
(87) International publication number: WO 1998/017808

(56) References cited:
- WO-A-95/00554
- WO-A-95/19175
- US-A- 5 270 458
- NAKAO M ET AL: "INTERNAL TANDEM DUPLICATION OF THE FLT3 GENE FOUND IN ACUTE MYELOIDLEUKEMIA" LEUKEMIA, MACMILLAN PRESS LTD, US, vol. 10, 1996, pages 1911-1918, XP002922821 ISSN: 0887-6924
- ROSSNER MICHAEL T ET AL: "Fms-like tyrosine kinase 3 catalytic domain can transduce a proliferative signal in FDC-P1 cells that is qualitatively similar to the signal delivered by c-Fms." CELL GROWTH & DIFFERENTIATION, vol. 5, no. 5, 1994, pages 549-555, XP001105308 ISSN: 1044-9523
- ROSNET OLIVIER ET AL: "Human FLT3/FLK2 gene: cDNA cloning and expression in hematopoietic cells." BLOOD, vol. 82, no. 4, 1993, pages 1110-1119, XP001105306 ISSN: 0006-4971
- ZEIGLER FRANCIS C ET AL: "Cellular and molecular characterization of the role of the FLK-2/FLT-3 receptor tyrosine kinase in hematopoietic stem cells." BLOOD, vol. 84, no. 8, 1994, pages 2422-2430, XP002212829 ISSN: 0006-4971
- DATABASE BIOSIS ON DIALOG, BIOSIS Number: 99755642, HORIKE S. et al., "Tandem Duplications of the FTL3 Receptor Gene Are Associated with Leukemic Transformation of Myeloidyplasia"; & LEUKEMIA, (BASINGSTOKE), 11(9), 1997, pages 1442-1446.
- DATABASE BIOSIS ON DIALOG, BIOSIS Number: 99364760, NANAO M. et al., "Internal Tandem Duplications of the FTL3 Gene Found in Acute Myeloid Leukemia"; & LEUKEMIA, (BASINGSTOKE), 10(12), 1996, pages 1911-1918.
- PROC. NATL. ACAD. SCI. U.S.A., 1995. Vol. 91, DONALD SMALL et al., "STK-1, the Human Homolog of Flk-2/Flk-3, is Selectively Expressed in CD34+ Human Bone Marrow Cells and is Involved in the Proliferation of Early Progenitor/Stem Cells", pages 459-463.
- CELL, 1991, Vol. 65, WILLIAM MATTHEWS et al., "A Receptor Tyrosine Kinase Specific to Hematopoietic Stem and Progenitor Cell-Enriched Populations", pages 1143-1152.

## Description

### TECHNICAL FIELD

The present invention concerns a nucleic acid encoding a receptor protein kinase, which has tandem duplication in a nucleotide sequence of a juxtamembrane, a polypeptide, a method for detection of the above nucleic acid and a kit for detection.

### BACKGROUND ART

Proliferation and differentiation of cells, and responses of cells to various stimuli are strictly regulated by various growth factors. These growth factors are known to act via receptors which are specific to the above growth factors (Nicola, N.A., *Annu. Rev. Biochem*. **58**, 45, 1989; Lowenberg, B., *Blood* **81,** 281, 1991). Of those receptors, the receptors containing a tyrosine kinase domain are classified as receptor tyrosine kinases (RTKs).

RTKs comprise an extracellular region, a transmembrane region, as well as an intracellular region containing a tyrosine kinase domain and a juxtamembrane between the transmembrane region and the tyrosine kinase domain, and further roughly classified into four types according to structural characteristics and amino acid sequence homology.

Type I receptors have a monomeric structure, with two cysteine-rich repeat sequences in their extracellular region, and are exemplified by the EGF receptor, HER2/neu and the like.

Type II receptors have a structure comprising two subunits each for α and β, which are bound via S-S bond, wherein the α chain is an extracellular region containing one cysteine-rich repeat sequence, and wherein the β chain has a transmembrane region, a juxtamembrane, and a tyrosine kinase domain. Examples are an insulin receptor and an IGF-1 receptor.

Type III receptors have a monomeric structure containing five immunoglobulin-like cysteine-rich sequences in their extracellular region and two tyrosine kinase domains interrupted by a kinase insert in their intracellular region. Examples are PDGF receptor, fms (CSF-1 receptor), kit (SLF receptor) and the like.

Type IV receptors resemble type III receptors but have three immunoglobulin-like repeat sequences, and are exemplified by FGF receptor.

fms-Like tyrosine kinase 3 (hereinafter abbreviated as FLT3; Matthews, W., *Cell* **65,** 1143, 1991; Rosnet, O., *Genomics* **9**, 380, 1991), which is expressed in leukemic cells etc., also referred to as fetal liver kinase 2 (FLK2) or STK-1, is known to as type III receptors (Small, *D., Proc. Natl. Acad. Sci. USA* **91,** 459, 1993; Lyman, S.D., *Oncogene* **8,** 815, 1993; Rosnet, O., *Blood* **82,** 1110, 1993; Agnes, F., *Gene* **145,** 283, 1994).

In these receptor tyrosine kinases, aggregation, such as dimerization, takes place upon binding of a ligand, such as a growth factor, to the extracellular region, thereby resulting in the activation of kinase. Although in these tyrosine kinases, their ligands have been first found and then their receptors in most cases, there are receptors of which ligands remain unknown.

Regarding FLT3, which has been remarked in proliferation mechanism of hematopoietic stem cells and leukemia, after finding the *FLT3,* the FLT3 ligand has been found (Lyman, S.D., *Cell* **75,** 1157, 1993; Hannum, C., *Nature* **368,** 643, 1994). Since the FLT3 ligand is expressed in almost all leukemic cells, it is assumed that cells are proliferated by a mechanism of autocrine stimulation in leukemia (Meierhoff, G., *Leukemia* **9,** 1368, 1995). Also, FLT3 mRNA has been reported to be expressed in lymphatic leukemic cells and myelocytic leukemic cells (Birg, F., *Blood* **80,** 2584, 1994; Da Silva, N., *Leukemia* **8,** 885, 1994; Brasel, K., *Leukemia* **9,** 1212, 1995; Drexler, H.G., *Leukemia* **10**, 588, 1996). However, there remains unknown how the FLT3 mRNA expression is associated with the pathology of lymphatic leukemia and myelocytic leukemia.

A human FLT3 cDNA has been cloned, and a cDNA nucleotide sequence and the amino acid sequence of the FLT3 protein have been determined [O. Rosnet et al., *Blood*, **82**(4), 1110 - 1119 (1993)]. The present situation, however, is that the structure and function of FLT during the hematopoietic stem cell differentiation and the malignant alterations to leukemic cells have not been analyzed well.

### DISCLOSURE OF INVENTION

Accordingly, a first object of the present invention is to provide a nucleic acid encoding a receptor protein kinase, wherein the nucleic acid has tandem duplication in a nucleotide sequence of a juxtamembrane and is useful for diagnosis of leukemia, and to provide a nucleic acid encoding the above juxtamembrane. A second object of the present invention is to provide a polypeptide which is encoded by the above nucleic acid. A third object of the present invention is to provide an antibody capable of specifically binding to a portion encoded by a nucleic acid having tandem duplication occurring in a nucleotide sequence of a juxtamembrane. A fourth object of the present invention is to provide a nucleic acid capable of specifically binding to a nucleic acid having tandem duplication occurring in a nucleotide sequence of a juxtamembrane. A fifth object of the present invention is to provide a method for detection of the nucleic acid encoding a receptor protein kinase and a kit therefor.

Conventionally, as to the FLT3, the same receptor protein kinase is expressed, irrespective of kinds of cells and differentiation [O. Rosnet et al., *Blood,* **82**(4), 1110 - 1119 (1993)]. As a result of the detailed investigation and intensive studies of the FLT3 expression in leukemic cells, however, the present inventors surprisingly have found a receptor protein kinase gene having novel tandem duplication in a juxtamembrane, and found that the above tandem duplication is somatic, and that the expression of FLT3 having the above tandem duplication is associated with leukemia malignancy and mal-consequence of prognosis, and the present invention has been completed thereby.

Accordingly, the gist of the present invention is as follows:
[1] a nucleic acid encoding a receptor protein kinase, wherein the nucleic acid has tandem duplication in a nucleotide sequence of a juxtamembrane;
[2] the nucleic acid according to item [1] above, wherein the receptor protein kinase is a receptor tyrosine kinase;
[3] the nucleic acid according to item [2] above, wherein the receptor tyrosine kinase is FMS-like tyrosine kinase 3 (FLT3);
[4] the nucleic acid according to any one of items [1] to [3] above, wherein the nucleic acid comprises a nucleotide sequence encoding an amino acid sequence as shown by any one of SEQ ID NOs: 1 to 5 in Sequence Listing in a juxtamembrane;
[5] the nucleic acid according to any one of items [1] to [3] above, wherein the nucleic acid comprises a nucleotide sequence as shown by any one of SEQ ID NOs: 6 to 15 in Sequence Listing in a juxtamembrane;
[6] a nucleic acid encoding a tandem duplication mutant of FLT3 as shown by any one of SEQ ID NOs: 16 to 20 in Sequence Listing;
[7] a nucleic acid comprising a nucleotide sequence encoding a tandem duplication mutant as shown by any one of SEQ ID NOs: 21 to 25 in Sequence Listing, or a nucleic acid capable of hybridizing thereto under stringent conditions, wherein the nucleic acid has tandem duplication in a nucleotide sequence encoding a juxtamembrane;
[8] a nucleic acid having tandem duplication, wherein the nucleic acid encodes an amino acid sequence as shown by any one of SEQ ID NOs: 1 to 5 in Sequence Listing;
[9] a nucleic acid as shown by any one of SEQ ID NOs: 6 to 15 in Sequence Listing, or a nucleic acid capable of hybridizing thereto under stringent conditions, wherein the nucleic acid has tandem duplication;
[10] a polypeptide encoded by the nucleic acid according to any one of items [1] to [9] above;
[11] a polypeptide comprising an amino acid sequence as shown by any one of SEQ ID NOs: 1 to 5, and 16 to 20 in Sequence Listing;
[12] a polypeptide encoded by a nucleic acid having tandem duplication in a nucleotide sequence of a juxtamembrane, wherein the polypeptide results from at least one of deletion, substitution or addition of one or more amino acid residues in an amino acid sequence as shown by any one of SEQ ID NOs: 1 to 5, and 16 to 20;
[13] an antibody capable of specifically binding to a region encoded by a nucleic acid having tandem duplication occurring in a nucleotide sequence of a juxtamembrane of a receptor protein kinase;
[14] a nucleic acid capable of specifically binding to a nucleic acid having tandem duplication occurring in a nucleotide sequence of a juxtamembrane of a receptor protein kinase;
[15] a method for detection of a nucleic acid encoding receptor protein kinase and having tandem duplication occurring in a nucleotide sequence of a juxtamembrane, comprising:
   step (a): preparing a human nucleic acid sample;
   step (b): subjecting the nucleic acid sample obtained in step (a) to gene amplification reaction to provide a nucleic acid fragment obtained by amplifying a region having tandem duplication in a juxtamembrane which can be found in a nucleic acid encoding a receptor protein kinase; and
   step (c): examining the presence of tandem duplication for the nucleic acid fragment of step (b);
[16] the method for detection according to item [15] above, characterized in that the method is utilized in diagnosis of M2, M4, or M5 based on the FAB (French-American-British) classification of acute myeloid leukemia;
[17] a kit for detection of a nucleic acid encoding a receptor protein kinase and having tandem duplication in the nucleotide sequence of a juxtamembrane, characterized in that the kit comprises primers for amplifying a region having tandem duplication, wherein the region can be found in the receptor protein kinase gene;
[18] the kit according to item [17] above, characterized in that the kit is utilized in diagnosis of M2, M4, or M5 based on the FAB (French-American-British) classification of acute myeloid leukemia; and
[19] use of the nucleic acid according to any one of items [1] to [9] above for detection of a nucleic acid encoding a receptor protein kinase and having tandem duplication in a nucleotide sequence of a juxtamembrane.

### BRIEF DESCRIPTION OF THE DRAWINGS

Figure 1 is a view showing agarose gel electrophoresis of the case where RT-PCR is carried out with RNA obtained from leukemic cells derived from AML patients as a template. In the figure, lanes 1 to 5 respectively show results for patients belonging to M1, M2, M3, M4 and M5 (M34 patients) on FAB classification, and lanes 6 to 9 respectively show results for M1, M2 (M155 patients), M3 and M4 (M162 patients).
Figure 2 is a schematic view showing tandem duplication at exon 11 and exon 12 for M34, M155, M162, M810 and M839.

### BEST MODE FOR CARRYING OUT THE INVENTION

The present invention will be explained below.

The nucleic acid encoding a receptor protein kinase of the present invention has tandem duplication in a region encoding a juxtamembrane. The nucleic acid of the present invention encoding a protein kinase is a nucleic acid encoding a tyrosine kinase, in particular an FMS-like tyrosine kinase 3 (FLT3). For diagnosis of leukemia nucleic acids encoding FMS-like tyrosine kinase 3 (FLT3) are used.

In the present invention, the juxtamembrane is present between the transmembrane region and the kinase domain of the receptor protein kinase, and the juxtamembrane constructs an intracellular membrane region together with the kinase domain [O. Rosnet, et al., *Blood,* **82** (4), 1110-1119 (1993)].

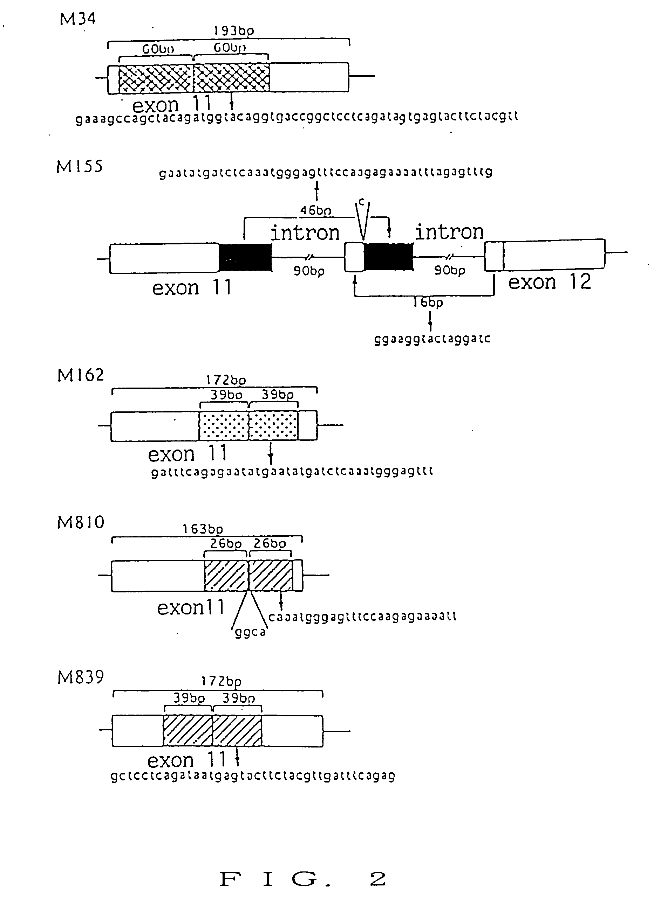

In the present invention, tandem duplication refers to an FLT3 nucleotide sequence in which an entire portion or partial portion of a nucleic acid encoding a juxtamembrane is repeated one or more times in the same orientation. The above repeat nucleotide sequences can be lined up one directly after another, or they can contain optional nucleotide sequences between each of the repeat nucleotide sequences. In addition, the number of duplicated base is not particularly limited. Furthermore, mutations of deletion, substitution or addition of one or more bases can exist in a portion of a nucleotide sequence between the corresponding tandem duplications. In the tandem duplication of the present invention, the tandem duplication may be detected as length mutation. For example, the tandem duplication is contained in cDNA having nucleotide sequences of SEQ ID NOs: 6 to 10, and in genomic DNA having nucleotide sequences of SEQ ID NOs: 11 to 15 as nucleic acids encoding a juxtamembrane. FLT3 nucleic acids (cDNA or genomic DNA) having tandem duplication which are newly found in a nucleotide sequence of the juxtamembrane of FLT3 are named M34 (SEQ ID NOs: 6 and 11), M155 (SEQ ID NOs: 7 and 12), M162 (SEQ ID NOs: 8 and 13), M810 (SEQ ID NOs: 9 and 14) and M839 (SEQ ID NOs: 10 and 15), respectively, and their schematic view is shown in Figure 2. Incidentally, it is desired that the tandem duplication-in the present invention takes place in-frame. Amino acid sequences encoded by the above SEQ ID NOs: 6 to 10 are shown in SEQ ID NOs: 1 to 5.

The nucleic acid of the present invention concerns a nucleic acid of FMS-like tyrosine kinase 3 (FLT3-) mutant, wherein the nucleic acid has the tandem duplication in a nucleotide sequence of a juxtamembrane. The amino acid sequences of a juxtamembrane having the tandem duplication are shown by e.g. SEQ ID NOs: 1 to 5 as mentioned above, and the nucleic acids of the present invention are those comprising FLT3 nucleotide sequences encoding these amino acid sequences. Concretely, for example, the present nucleic acids are those comprising nucleotide sequences shown by SEQ ID NOs: 6 to 15. More particularly, a nucleic acid of a tandem duplication mutant of FLT3 comprising a nucleic acid of a juxtamembrane includes, for example, nucleic acids encoding tandem duplication mutants of FLT3 shown by SEQ ID NOs: 16 to 20, more concretely, nucleic acids comprising nucleotide sequences encoding tandem duplication mutants of FLT3 shown by SEQ ID NOs: 21 to 25. In addition, they may be a nucleic acid capable of hybridizing to the above nucleic acid under stringent conditions, and having tandem duplication in a nucleotide sequence encoding a juxtamembrane.

Furthermore, the nucleic acid of the present invention concerns an FLT3 nucleic acid encoding a juxtamembrane and having tandem duplication, the nucleic acid including, for example, a nucleic acid having tandem duplication, wherein the nucleic acid encodes amino acid sequences shown by SEQ ID NOs: 1 to 5, concretely, nucleic acids shown by SEQ ID NOs: 6 to 15, or a nucleic acid has tandem duplication capable of hybridizing to those nucleic acids under stringent conditions.

Here, hybridizing under stringent conditions refers to hybridization with the nucleic acids, wherein the hybridization comprises, for example, incubating a nucleic acid-immobilized membrane with a probe at 50°C for 12 to 20 hours in 6 x SSC, wherein 1 x SSC indicates 0.15 M NaCl, 0.015 M sodium citrate, pH 7.0, containing 0.5% SDS, 0.1% bovine serum albumin (BSA), 0.1% polyvinyl pyrrolidone, 0.1% Ficol 400, and 0.01% denatured salmon sperm DNA, but not limited to the above conditions.

The nucleic acid of the present invention can be obtained by, e.g. the following method.

First, cells in which length mutation takes place are detected by synthesizing cDNA by a reverse transcriptase with RNA as a template, the RNA purified from various pathologic cells, particularly leukemia cells, thereafter carrying out DNA amplification reaction using primers which are targeted to a region encoding a juxtamembrane of a desired receptor protein kinase, and comparing the length of the amplified DNA fragments by means of an electrophoresis method. Further, it is possible to identify whether or not a mutation is tandem duplication by determining a nucleotide sequence of the obtained amplified DNA fragment.

Next, cDNA encoding a receptor protein kinase, the cDNA having novel tandem duplication of the present invention can be obtained by synthesizing cDNA by a reverse transcriptase with RNA obtained from cells in which tandem duplication takes place, thereafter carrying out DNA amplification reaction using primers which can specifically amplify cDNA of a desired receptor protein kinase.

The nucleic acid of the present invention can be also obtained by using genomic DNA as a template, the genomic DNA purified from pathologic cells.

In the present invention, leukemia cells are selected as the pathologic cells, and FLT3 is preferably targeted as the receptor protein kinase.

An FLT3 gene comprises 21 exons, and alternatively, the juxtamembrane is encoded in 18 bp at 3'-side of exon 10 and 117 bp at 5'-side of exon 11 [O. Rosnet, et al., *Oncogene,* 6, 1641 - 1650 (1991)]. Primers covering the region of exon 11 and exon 12 can be selected as primers used in DNA amplification reaction. Examples of the nucleotide sequences are shown in SEQ ID NOs: 26 and 27. Incidentally, exon 12 and 16 bp at 3'-side of exon 11 encode a partial portion of the tyrosine kinase domain.

DNA amplified fragments as shown by SEQ ID NOs: 6 to 10 are obtained when RNA is used as a template for DNA amplification reaction, and DNA amplified fragments as shown by SEQ ID NOs: 11 to 15 are obtained when genomic DNA is used as a template. As a result, it is confirmed that these resulting fragments have in-frame tandem duplication within exon 11 or exons 11 to 12.

Alternatively, nucleotide sequences of cDNA encoding a whole length of FLT3 and having the above in-frame tandem duplication are shown in SEQ ID NOs: 21 to 25.

The polypeptide of the present invention is a polypeptide encoded by the above nucleic acids. Concretely, there can be exemplified a polypeptide comprising amino acid sequences of SEQ ID NOs: 1 to 5, and tandem duplication mutants of FLT3 as shown by SEQ ID NOs: 16 to 20.

The polypeptide of the present invention can be obtained by purifying from cells expressing the polypeptide, and can be also obtained by employing a conventional gene engineering procedures. A tandem duplication mutant of FLT3, for example, can be obtained by inserting the above nucleic acids into a suitable expression vector, and then expressing the product in a suitable host. In addition, a polypeptide with only a juxtamembrane of a receptor protein kinase of the present invention can be obtained by inserting a DNA fragment encoding a juxtamembrane alone to the above expression vector.

Further, the polypeptide of the present invention can be expressed as a fusion protein. For instance, in order to increase amounts of expression of a desired protein, N-terminal peptide chain derived from other protein is added to N-terminus of the desired protein, or a suitable peptide chain is added to N-terminus or C-terminus of the desired protein to express the resulting polypeptide, so that purification of the desired protein using a resin carrier having affinity to the peptide chain can be facilitated.

The present polypeptide also encompasses a polypeptide encoded by a nucleic acid having tandem duplication in a nucleotide sequence of a juxtamembrane, wherein the polypeptide results from at least one of deletion, substitution or addition of one or more amino acid residues in amino acid sequences of the present invention, e.g. SEQ ID NOs: 1 to 5, 16 to 20 in Sequence Listing. In other words, there can be a case where no mutations take place in amino acid sequences in the region encoded by tandemly duplicated nucleic acids, and deletion, substitution or addition of amino acid residues takes place in other portions of amino acid sequences; or a case where deletion, substitution or addition of amino acid residues takes place in amino acid sequences of the region encoded by tandemly duplicated nucleic acids. Introduction of deletion, substitution or addition of the amino acid residues can be easily carried out by introducing mutation into the desired nucleic acid sequence by a method using restriction endonucleases, nucleases and the like, or a method for performing site-directed mutagenesis [W. Ito, et al., *Gene,* **102**, 67 - 70 (1991)] etc., thereby incorporating the mutated nucleic acid sequence into an expression vector to express the product in a suitable host cell.

In the present invention, the antibody refers to an antibody capable of specifically binding to a region encoded by a nucleic acid having tandem duplication occurring in a nucleotide sequence of a juxtamembrane of the receptor protein kinase. In order to obtain the antibody, for example, the antibody is obtained as antiserum by immunizing animals with a peptide having amino acid sequences of SEQ ID NOs: 1 to 5 together with adjuvant by conventional method. In addition, the antibody can be obtained as a monoclonal antibody by a method described in G. Galfare, et al., *Nature*, ·**266**, 550 - 552 (1997).

In the present invention, the nucleic acid capable of specifically binding to nucleic acids having tandem duplication occurring in a nucleotide sequence of a juxtamembrane of the receptor protein kinase is not to be particularly limited, and is exemplified by antisense DNA of double stranded DNA having tandem duplication or RNA corresponding to the antisense DNA.

The method for detection of a nucleic acid of the present invention comprises the following steps:
step (a): obtaining a human nucleic acid sample;
step (b): subjecting the nucleic acid sample obtained in the above step (a) to gene amplification reaction to provide a nucleic acid fragment obtained by amplifying a region having tandem duplication in a juxtamembrane, wherein the region can be found in a nucleic acid encoding a receptor protein kinase; and
step (c): examining the presence of tandem duplication for the nucleic acid fragment of the above step (b).

First, step (a) will be described. The human nucleic acid sample usable in the present invention is not to be particularly limited, as long as it is a nucleic acid encoding a receptor protein kinase, the nucleic acid having tandem duplication in a nucleotide sequence of a juxtamembrane, such as genomic DNA, cDNA or mRNA. The human nucleic acid sample can be prepared by conventionally performed known method, including, for instance, a method described in Molecular *Cloning:* A *LABORATORY MANUAL,* 2nd eds. (T. Maniatis et al., Cold Spring Harbor Laboratory Press, published in 1989).

Secondly, step (b) will be described. The nucleic acid sample and suitable primers are used to amplify a nucleic acid encoding a region containing mutation site which can be found in a juxtamembrane of the receptor protein kinase of the interest to obtain a desired nucleic acid fragment. A method for performing DNA amplification reaction usable in this step is not particularly limited, as long as it is a method capable of amplifying the above region, and there can be utilized nucleic acid amplification methods, such as a nucleic acid amplification method utilizing RT-PCR method, PCR method, or RNA polymerases (Japanese Patent Laid-Open Nos. Hei 2-5864 and Hei 7-203999), or strand substitution amplification method (Japanese Examined Patent Publication No. Hei 7-114718, and Japanese Patent Laid-Open No. Hei 7-88242). Among them, the RT-PCR method or PCR method is preferably used.

The region to be amplified having tandem duplication in a juxtamembrane includes, for example, in case of FLT3, a region containing a whole or partial portion of the region from 18 bp at 3'-side of exon 10 to 117 bp at 5'-side of exon 11, without being particularly limited thereto as long as the region contains an exon 11 site.

The primers used in RT-PCR method or PCR method are not particularly limited as long as they are primers capable of amplifying a DNA fragment containing the above mutation site. Concretely, there can be exemplified a primer pair having nucleotide sequences as shown in SEQ ID NOs: 26 and 27 in Sequence Listing. In addition, PCR conditions are not particularly limited, and conventionally performed known conditions can be used on PCR reaction.

Thirdly, step (c) will be described. In this step, the presence of tandem duplication for the nucleic acid fragment obtained in step (b) is examined. The method for detection of the presence of tandem duplication is not particularly limited, and it is preferable that a method of comparing lengths of amplified DNA fragments by means of agarose gel electrophoresis method is used.

In addition, a method for examining single strand conformation polymorphism (SSCP) can be used as a method for detection of mutation which is usable in this step. The method comprises examining the differences of a higher-order structure as the differences of mobility in electrophoresis, wherein the high-order structure is dependent on a nucleotide sequence in which single-stranded DNA is formed by intramolecular interaction (*Proc. Natl. Acad. Sci. USA,* **86**: 2766 - 2770, 1989). The presence or absence of mutation can be detected by subjecting the nucleic acid fragment obtained in step (b) to electrophoresis under conditions described in the above-mentioned publication, and comparing its mobility with that of a nucleic acid fragment derived from a normal receptor protein kinase.

Other detection methods include a method in which the above step (c) is altered to other method for detection of mutation. For the detection of mutation, there can be used a known method for detection of mutation, such as hybridization method using a suitable DNA fragment containing a mutation site as a probe, or DGGE method [Val C. Sheffield et al., *Proc. Natl. Acad. Sci. USA* **86**, 232 - 236 (1989)]. In addition, a method for detection of mutation using a MutS protein is known (Japanese Patent Laid-Open No. Hei 7-327698).

The mutation can be identified by sequencing the nucleotide sequence for DNA fragment in which a length mutation is confirmed by means of the above-mentioned method. For sequencing the nucleotide sequence, a conventionally used method can be employed, including, for example, a method comprising cloning an amplified DNA fragment into a suitable vector and determining the nucleotide sequence, or a method for determining the nucleotide sequence using an amplified fragment per se as a template.

As described above, the present invention provides a use of a nucleic acid of the present invention described above for detection of a nucleic acid encoding a receptor protein kinase, wherein the nucleic acid has tandem duplication in a nucleotide sequence of a juxtamembrane.

The method for detection of a nucleic acid of the present invention can be utilized in diagnosis of M2, M4, and M5 based on the FAB (French-American-British) classification of acute myeloid leukemia (AML). Based on the FAB (French-American-British) classification, pathologic types of AML are classified into six classes as M1 (myeloblastic, no maturation potential), M2 (myeloblastic, with maturation potential), M3 (promyelocytic), M4 (myelomonocytic), M5 (monocytic), and M6 (erythroleukemia) (Shin Rinsho Kensa Gishi Koza, 10, *Ketsuekigaku,* 75, Igaku-Shoin).

It is understood that patients harboring an FLT3 gene having the tandem duplication of the present invention belong to classes M2, M4, and M5 above, and that the patients relapse into symptom to death even with transient symptom remission, so that their prognosis leads to mal-consequence. Therefore, according to the detection method of the present invention, there can be provided a method for examination useful to the pathologic judgment of AML.

Incidentally, of the above patients, the detection of mutation using genomic DNA from myelocyte of a patient obtained at the time of symptom remission is carried out, and as a result, tandem duplications in a juxtamembrane are not found. This mutation is therefore assumed to be a somatic mutation.

Also, the nucleic acid of the present invention, which has tandem duplication, serves as a marker for myelodysplastic syndrome (MDS), which develops in the pre-stage of leukemia, AML with dysplasia, and the like, as well as AML as classified based on the FAB classification. The detection method of the present invention therefore is a method for examination which is useful for the pathologic judgment of these diseases.

By utilizing the above detection method, there can be provided a kit for detection of a nucleic acid of the present invention. Concretely, there is a kit for detection of a nucleic acid by the above described detection method, the nucleic acid encoding a receptor protein kinase and having tandem duplication in the nucleotide sequence of a juxtamembrane, characterized in that the kit comprises primers for amplifying a region having tandem duplication, wherein the region can be found on the receptor protein kinase gene.

The diagnosis of the above AML etc. can be easily carried out by using such a kit.

In the present invention, a polypeptide encoded by a nucleic acid having tandem duplication as described above can be further detected by the steps shown below:
step 1: obtaining a human protein sample; and
step 2: examining the presence of tandem duplication in the nucleotide sequence of a juxtamembrane of the protein sample obtained in the above step 1.

First, step 1 will be described. The human protein sample can be prepared by preparing a membrane protein from a cell which is assumed to have the polypeptide of the present invention expressed therein (e.g., leukemic cell, in case of FLT3).

Second, step 2 will be described. The method for detection of tandem duplication mutations is not particularly limited, and can be carried out by using a labeled antibody capable of specifically binding to the juxtamembrane encoded by a nucleic acid having a tandem duplication mutation.

This step can, for example, be carried out by a method comprising subjecting the protein sample obtained in step 1 to SDS-PAGE to separate proteins, and subsequently detecting the desired protein by immunoblotting method.

In another embodiment of the present invention, there can be provided a method for regulating the proliferation, immune response and signal information transmission of leukemic cells, hematopoietic stem cells, etc. using the above nucleic acids or polypeptides, or nucleic acids or antibodies capable of specifically binding thereto.

Among them, a preferred embodiment includes an application to immunotherapy for tumors. Conventionally, it has been known that tumor-specific peptides of proteins specifically expressed in tumor cells serve as targets of T cell immune responses to tumor cells. In a method for performing the application, the techniques described in the following reports, for example, can be utilized. Concretely, CD4+T cells restricted to HLA-DR are isolated, the cells specifically reacting with ras peptide resulting from substitution of 12th amino acid glycine with another amino acid in the human T cells (Jung, S., *J*. *Exp. Med. 173,* 273, 1991), and a CTL (cytotoxic T lymphocyte) recognizing a peptide consisting of eight amino acids including the mutation site for 61th amino acid mutation can be derived from a mouse immunized with a recombinant vaccinia virus capable of producing ras protein, which has mutation at 61th amino acid (Skipper, J., *J. Exp. Med. 177,* 1493, 1993). In addition, in a mouse immunized with a soluble mutant ras protein prepared by gene recombination, the *in vivo* proliferation of tumor cells having the same mutation is suppressed (Fenton, R.G., *J*. *Natl. Cancer Inst.* **85**, 1294, 1993), and a CTL showing cytotoxic activity against tumor cells expressing the same mutant ras can be obtained from splenocytes sensitized with the mutant ras peptide (Peace, D.J., *J. Exp. Med*. **179**, 473, 1994). On the other hand, the bcr-abl chimeric protein, which is often detected in chronic myelocytic leukemia, possesses high tyrosine kinase activity and plays a key role in the onset of leukemia and the proliferation of leukemic cells. By immunizing with a peptide in the vicinity of the fusion site of this fusion protein, T cells reactive to this fusion protein can be obtained (Chen, W., *Proc. Natl. Acad. Sci. USA* **89**, 1468, 1992). Moreover, antisense DNA or RNA corresponding to the fusion gene is capable of suppressing the proliferation of tumors expressing this gene *in vivo* (Skorski, T., *Proc. Natl.* Acad. *Sci. USA* **91**, 4504, 1994).

It is therefore possible to obtain T cells reactive to a receptor protein kinase comprising the peptide of the present invention, wherein the peptide is encoded by a nucleic acid having tandem duplication occurring in the nucleotide sequence of a juxtamembrane, and to regulate the proliferation of cells that express the above kinase by immunizing with the above peptide.

Also, when the presence of the tandem duplication of the present invention is involved in cell proliferation regulation, it is possible to regulate the signaling mechanism with antisense DNA or RNA for the above gene to regulate cell proliferation.

When binding a ligand to an extracellular region, the receptor protein kinase undergoes a conformational change to form a dimer, resulting in increased kinase domain activity in the intracellular region, whereby self-phosphorylation or phosphorylation of a substrate of the above kinase takes place. In these steps, various signaling molecules are involved, and the information transmitted into cells causes various biological phenomena, such as cell morphological change, cell movement, morphogenesis, cell proliferation, malignant alteration, differentiation, and apoptosis. Acute myelocytic leukemic cells of high malignancy have been reported to possess strong affinity to the FLT3 ligand and promote cell proliferation (Piacibello, W., *Blood* **86**, 4105, 1995; Lisovsky, M., *Blood* **86**, 22a, 1995; McKenna, H., *J*. *Exp*. *Hematol.* **24**, 378, 1996; Dehmel, U., Leukemia 10, 261, 1996). In cells expressing the FLT3 tandem duplication mutant of the present invention, it is expected that the system for signaling from the FLT3 ligand is highly activated. Hematopoietic stem cells that express the mutant are therefore provided as a source of hematopoietic stem cells possessing strong proliferation potential. By comparing the hematopoietic stem cells with cells expressing the normal FLT3, materials and methods suitable for screening for various drugs can be provided.

As described above, by utilizing a method of the present invention, it is applicable to the examination and treatment of blood cell diseases, hematopoietic stem cell diseases, and other diseases.

The present invention will be hereinafter described in more detail by means of examples, but the present invention is not limited by those examples.

### Example 1

### 1) Analysis of FLT3 gene expression pattern

On 80 cases of acute leukemia patients (50 cases of child ALL, 30 cases of adult AML), analysis of FLT3 gene expression was carried out by RT-PCR method. The primers used were designed to have nucleotide sequences as shown by SEQ ID NOs: 26 and 27 in Sequence Listing, and to completely cover and amplify a transmembrane region through a juxtamembrane. By using the above primer pair, the resulting amplified DNA product is 366 bp in length, when normal FLT3 has been transcribed.

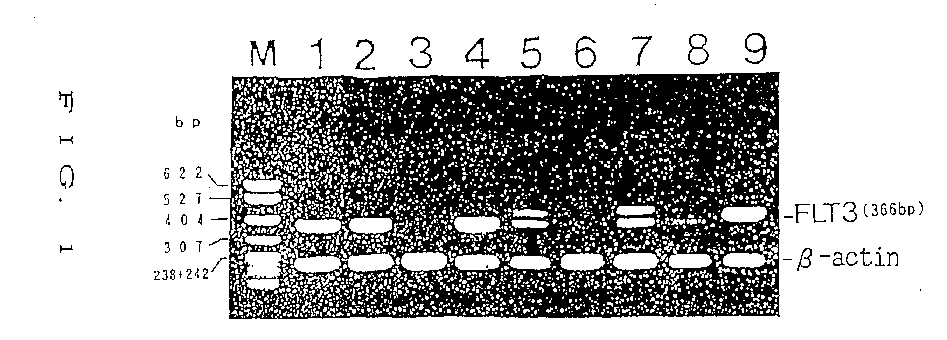

A total RNA was extracted from a peripheral blood or myelocyte derived from the above patient with a Trizol reagent (manufactured by LifeTech), followed by DNA amplification reaction using an RT-PCR kit (manufactured by Takara Shuzo Co., Ltd.) and Thermal Cycler (manufactured by Takara Shuzo Co., Ltd.) under following conditions. cDNA was synthesized from a total RNA using a reverse transcriptase. In 50 µl of a reaction mixture containing 1 µl of the cDNA (equivalent to 40 ng of a total RNA), 200 µM dNTP mixture, 1 x PCR buffer, 2 U of Taq DNA polymerase, and 20 pmol each of the above-described primers, the above reaction mixture was heated at 94°C for 5 minutes, and thereafter repeated 35 times of a thermal cycle consisting of 64°C for 30 seconds, 72°C for 45 seconds, and 94°C for 30 seconds, and then finally treated at 72°C for 5 minutes. To check quality of RNA, RT-PCR was carried out in the same manner except that a pair of the primers shown by SEQ ID NOs: 28 and 29 in Sequence Listing were used, with P-actin as the target. The amplified DNA products thus obtained were subjected to electrophoresis on 2 to 3% agarose gel (manufactured by FMC) containing ethidium bromide, and detected under UV irradiation. One example of electrophoresis pattern is shown in Figure 1, and the results are shown in Table 1.

**Table 1**

| FAB subtype | Number of cases examined | Number of positive mRNA expression of FLT3 (%) | Length mutation (%) |
|---|---|---|---|
| AML total | 30 | 22 (73%) | 5 (17%) |
| M1 | 3 | 2 (67%) | 0 |
| M2 | 9 | 7 (78%) | 1 (14%) |
| M3 | 8 | 5 (63%) | 0 |
| M4 | 5 | 4 (80%) | 2 (40%) |
| M5 | 4 | 3 (75%) | 2 (50%) |
| M6 | 1 | 1(100%) | 0 |
| ALL total | 50 | 39 (78%) | 0 |
| CALL | 27 | 24 (89%) | 0 |
| pre-B ALL | 13 | 11 (85%) | 0 |
| B-ALL | 1 | 0 | 0 |
| T-ALL | 9 | 4 (44%) | 0 |

It is found from Table 1 that the transcription product of the FLT3 gene was found in 39 cases (78%) of the 50 ALL cases and 22 cases (73%) of the 30 AML cases. Among them, the amplified DNA product longer than the expected 366 bp was detected in 5 cases (23%) of the 22 FLT3-positive AML cases, so that a length mutation in FLT3 gene was observed. Incidentally, in four cases (M34, M155, M810, and M839), the expected 366 bp band and a longer band than the expected were detected. In one case (M162), the 366 bp band was not detected, and a longer band alone was detected.

### 2) Analysis of nucleotide sequence of length mutation product of FLT3 gene

To examine in more detail length mutations in the gene in the above 5 cases, the amplified DNA product was purified from agarose gel, and nucleotide sequences of the exon 11 and exon 12 regions were determined. As a result, it was confirmed that these length mutations resulted from tandem duplications in nucleotide sequences of the respective juxtamembrane. Concretely, a 39 bp or 60 bp tandem duplication within exon 11 was found in cases M34, M162, and M839; and a 26 bp tandem duplication including a 4 bp (GGCA) insert was found in case M810. In addition, case M155 was found to have a 63 bp tandem duplication comprising the first 16 bp of exon 12 immediately after exon 11, one cytosine residue insert, and the last 46 bp of exon 11. The nucleotide sequences obtained are shown in SEQ ID NOs: 6 to 10 in Sequence Listing, and a schematic view of these tandem duplications is shown in Figure 2.

Characteristically, these tandem duplications occur in-frame, and these mutations are reflected in the actually expressed polypeptides. The amino acid sequences encoded by these nucleotide sequences are shown in SEQ ID NOs: 1 to 5.

### 3) Analysis of nucleotide sequence of genomic DNA

Amplified DNA products obtained by PCR with FLT3 genomic DNA derived from myelocytes from the above 5 cases of patients as templates were analyzed. PCR reaction was carried out under amplification conditions such that 2 U of Taq DNA polymerase (Takara Shuzo Co., Ltd.) was added to a PCR buffer containing 50 ng of genomic DNA, 200 µM of a dNTP mixture, and 20 pmol of each of primers, to make up a total volume of 50 µl. For exons 10 to exon 19, each exon was individually subjected to amplification DNA reaction. The length mutation was observed in same manner as that when mRNA was analyzed in the case where exon 11 and exon 12 were amplified with primers of SEQ ID NOs: 30 and 31, and primers of SEQ ID NOs: 32 and 33 in Sequence Listing as pairs. When the PCR products were purified using QIAEXII (QIAGEN), cloned into pCRII vector (Invitrogen), and subjected to nucleotide sequence analysis, similar results to those with the nucleotide sequences of cDNA (SEQ ID NOs: 21 to 25) were obtained. The results are collectively shown in Figure 2.

### Example 2

### Analysis of mutations in juxtamembrane of receptor protein kinase and their pathologic relationship

To analyze mutations in a juxtamembrane of the receptor protein kinase and their pathologic relationship, the relationship between the pathologic classification of symptoms and FLT3 gene is shown in Table 1. Five cases showing tandem duplication in the nucleotide sequence of a juxtamembrane belonged to M2 (myeloblastic, with maturation potential), M4 (myelomonocytic), or M5 (monocytic) based on the FAB classification, and all of them were cases in which patients relapse into symptom to death even with transient symptom remission.

The nucleotide sequences of exon regions encoding tyrosine kinase domain were also analyzed, and no mutations were found in these regions.

Therefore, it was suggested that the in-frame tandem duplications in gene region encoding a juxtamembrane of FLT3 were associated with AML with monocyte growth was suggested.

Also, since such length mutations were not detected in DNA samples from myelocytes collected from three cases (M34, M162, and M810) at the time of complete remission, the tandem duplication of the present invention was found to be a somatic mutation.

### INDUSTRIAL APPLICABILITY

According to the present invention, there can be provided a novel receptor protein kinase having tandem duplication mutation in the nucleotide sequence of a juxtamembrane, and its nucleotide sequence and amino acid sequence information. In addition, there can be provided pathological diagnoses, a method for examination of leukemia etc. utilizing the present invention, a kit and a reagent for examination related thereto. Furthermore, there can be provided a method for regulating and analyzing conditions of proliferation and differentiation, malignant alteration, immune response, and signalling for cells represented by hematopoietic stem cells and leukemia cells utilizing the present invention, and a kit and a reagent related thereto.

## Claims

1. A nucleic acid molecule of a tandem duplication mutant encoding FMS-like tyrosine kinase 3 (FLT3), wherein said nucleic acid molecule has a nucleotide sequence corresponding to:
(a) a tandem duplication mutation in the amino acid sequence of juxtamembrane of FLT3, or
(b) a tandem duplication mutation in the nucleotide sequence of a region comprising exon 11 or exons 11 to 12 of FLT3.

2. A nucleic acid molecule having a nucleotide sequence corresponding to:
(a) a tandem duplication mutation in the amino acid sequence of juxtamembrane of FLT3, or
(b) a tandem duplication mutation in the nucleotide sequence of a region comprising exon 11 or exons 11 to 12 of FLT3.

3. A nucleic acid molecule capable of specifically hybridizing under stringent conditions to a nucleic acid molecule corresponding to:
(a) a tandem duplication mutation in the amino acid sequence of juxtamembrane of FLT3, or
(b) a tandem duplication mutation in the nucleotide sequence of a region comprising exon 11 or exons 11 to 12 of FLT3 in the nucleic acid of claim 1.

4. A polypeptide encoded by the nucleic acid molecule of claim 1, or the nucleic acid molecule of claim 2.

5. A cell expressing the nucleic acid molecule of claim 1, or the nucleic acid molecule of claim 2, or the polypeptide of claim 4.

6. An antibody which is specific for the polypeptide of claim 4.

7. A method for detecting the nucleic acid molecule of claim 1, or the nucleic acid molecule of claim 2, comprising the steps of:
(a)subjecting a nucleic acid sample from a human to a gene amplification reaction, wherein a nucleic acid fragment comprising exon 11 or exons 11 to 12 of the FMS-like tyrosine kinase 3 (FLT3) gene and having a tandem duplication mutation in the juxtamembrane is amplified, which can be found in FLT3 gene;
(b)detecting the presence of the tandem duplication mutation in the nucleic acid fragment of step (a).

8. The method of claim 7, wherein step (b) is carried out by comparing the amplified nucleic acid fragment obtained in step (a) with a sequence derived from a normal FLT3, thereby detecting the presence of tandem duplication mutation in the juxtamembrane.

9. The method of claim 7 or 8, wherein in step (b), a length mutation is used as an index of the tandem duplication mutation.

10. The method of any one of claims 7 to 9, wherein the gene amplification reaction in step (a) is carried out with a primer pair selected from the group consisting of: SEQ ID NOs: 26 and 27, SEQ ID NOs: 30 and 31 , and SEQ ID NOs: 32 and 33.

11. The method of any one of claims 7 to 10, wherein the tandem duplication mutation is not found in a gene of a normal individual.

12. A kit for the method of any one of claims 7 to 11, wherein said kit comprises primers for amplifying a region comprising exon 11 or exons 11 to 12 of the FLT3 gene.

13. The kit of claim 12, wherein said primers are selected from the group consisting of: SEQ ID NOs: 26 and 27, SEQ ID NOs: 30 and 31, and SEQ ID NOs: 32 and 33.

14. Use of a hematopoietic stem cell expressing a nucleic acid molecule encoding a tandem duplication mutant polypeptide and a cell expressing the normal FLT3 for screening a drug for examination and treatment of a blood cell disease or a hematopoietic stem cell disease.

15. The use of claim 14, wherein said nucleic acid molecule has a tandem duplication mutation in a nucleotide sequence of a juxtamembrane.

16. Use of a hematopoietic stem cell expressing the nucleic acid molecule of claim 1, or the nucleic acid molecule of claim 2, or the polypeptide of claim 4 and a cell expressing the normal FLT3 for screening a drug for examination and treatment of a blood cell disease or a hematopoietic stem cell disease.

17. Use of the nucleic acid molecule of claim 1 , or the nucleic acid molecule of claim 2 for the preparation of a drug for regulating proliferation, immune response or signal information transmission of a blood cell or a hematopoietic stem cell.

18. Use of the nucleic acid molecule of claim 1, or the nucleic acid molecule of claim 2, or polypeptide of claim 4, for the preparation of material used for pathologic judgment of myelodysplastic syndrome (MDS) or leukemia.

19. A pharmaceutical composition comprising the nucleic acid molecule of claim 1, and/or the nucleic acid molecule of claim 2, and/or the nucleic acid molecule of claim 3, and/or the polypeptide of claim 4, and/or the antibody of claim 12 and, optionally, a pharmaceutically acceptable carrier and/or diluent.

20. A kit for detection of the polypeptide of claim 4, wherein the kit comprises the antibody of claim 6.

## Patentansprüche

1. Nucleinsäuremolekül einer Tandemverdopplungsmutante, das FMS-artige Tyrosinkinase 3 (FLT3) codiert, wobei das Nucleinsäuremolekül eine Nucleotidsequenz hat entsprechend:
(a) einer Tandemverdopplungsmutation in der Aminosäuresequenz der Juxtamembran von FLT3, oder
(b) einer Tandemverdopplungsmutation in der Nucleotidsequenz einer Region umfassend Exon 11 oder Exons 11 bis 12 von FLT3.

2. Nucleinsäuremolekül mit einer Nucleinsäuresequenz entsprechend:
(a) einer Tandemverdopplungsmutation in der Aminosäuresequenz der Juxtamembran von FLT3, oder
(b) einer Tandemverdopplungsmutation in der Nucleotidsequenz einer Region umfassend Exon 11 oder Exons 11 bis 12 von FLT3.

3. Nucleinsäuremolekül, das fähig ist zur spezifischen Hybridisierung unter stringenten Bedingungen an ein Nucleinsäuremolekül entsprechend:
(a) einer Tandemverdopplungsmutation in der Aminosäuresequenz der Juxtamembran von FLT3, oder
(b) einer Tandemverdopplungsmutation in der Nucleotidsequenz einer Region umfassend Exon 11 oder Exons 11 bis 12 von FLT3 in der Nucleinsäure nach Anspruch 1.

4. Polypeptid, das von dem Nucleinsäuremolekül nach Anspruch 1 oder dem Nucleinsäuremolekül nach Anspruch 2 codiert wird.

5. Zelle, die das Nucleinsäuremolekül nach Anspruch 1 oder das Nucleinsäuremolekül nach Anspruch 2 oder das Polypeptid nach Anspruch 4 exprimiert.

6. Antikörper, der spezifisch ist für das Polypeptid nach Anspruch 4.

7. Verfahren zum Nachweis des Nucleinsäuremoleküls nach Anspruch 1 oder des Nucleinsäuremoleküls nach Anspruch 2, umfassend die Schritte:
(a) Durchführung einer Genamplifikationsreaktion mit einer Nucleinsäureprobe von einem Menschen, wobei ein Nucleinsäurefragment, das Exon 11 oder Exons 11 bis 12 des FMS-artigen Tyrosinkinase 3 (FLT3)-Gens umfasst und eine Tandemverdopplungsmutation in der Juxtamembran hat, amplifiziert wird, welches im FLT3-Gen gefunden werden kann;
(b) Nachweis der Anwesenheit der Tandemverdopplungsmutation in dem Nucleinsäurefragment aus Schritt (a).

8. Verfahren nach Anspruch 7, wobei Schritt (b) ausgeführt wird durch Vergleichen des amplifizierten Nucleinsäurefragments, erhalten in Schritt (a), mit einer Sequenz, die von einer normalen FLT3 abgeleitet ist, wobei auf diese Weise die Anwesenheit einer Tandemverdopplungsmutation in der Juxtamembran nachgewiesen wird.

9. Verfahren nach Anspruch 7 oder 8, wobei in Schritt (b) eine Längenmutation als Index für die Tandemverdopplungsmutation verwendet wird.

10. Verfahren nach einem der Ansprüche 7 bis 9, wobei die Gen-Amplifikationsreaktion in Schritt (a) mit einem Primerpaar ausgeführt wird, ausgewählt aus der Gruppe bestehend aus: SEQ ID NOs: 26 und 27, SEQ ID NOs: 30 und 31 und SEQ ID NOs.: 32 und 33.

11. Verfahren nach einem der Ansprüche 7 bis 10, wobei die Tandemverdopplungsmutation nicht in einem Gen einer normalen Testperson gefunden wird.

12. Kit für ein Verfahren gemäß einem der Ansprüche 7 bis 11, wobei der Kit Primer für die Amplifikation einer Region, die Exon 11 oder Exons 11 bis 12 des FLT3-Gens umfasst.

13. Kit nach Anspruch 12, wobei diese Primer ausgewählt sind aus der Gruppe bestehend aus: SEQ ID NOs: 26 und 27, SEQ ID NOs: 30 und 31 und SEQ ID NOs: 32 und 33.

14. Verwendung einer blutbildenden Stammzelle, die ein Nucleinsäuremolekül exprimiert, das ein Tandemverdopplungsmutations-Polypeptid codiert, und einer Zelle, die das normale FLT3 exprimiert, zur Durchmusterung auf einen Arzneistoff zur Untersuchung und Behandlung einer Blutzellerkrankung oder einer Erkrankung blutbildender Stammzellen.

15. Verwendung nach Anspruch 14, wobei dieses Nucleinsäuremolekül eine Tandemverdopplungsmutation in einer Juxtamembran-Nucleotidsequenz hat.

16. Verwendung einer blutbildenden Stammzelle, die das Nucleinsäuremolekül nach Anspruch 1 oder das Nucleinsäuremolekül nach Anspruch 2 oder das Polypeptid nach Anspruch 4 exprimiert, und einer Zelle, die das normale FLT3 exprimiert, zur Durchmusterung auf einen Arzneistoff zur Untersuchung und Behandlung einer Blutzellerkrankung oder einer Erkrankung blutbildender Stammzellen.

17. Verwendung des Nucleinsäuremoleküls nach Anspruch 1 oder des Nucleinsäuremoleküls nach Anspruch 2 für die Herstellung eines Arzneimittels zur Regulation der Proliferation, der Immunantwort oder der Signalinformationsübertragung einer Blutzelle oder einer blutbildenden Stammzelle.

18. Verwendung des Nucleinsäuremoleküls nach Anspruch 1 oder des Nucleinsäuremoleküls nach Anspruch 2 oder des Polypeptids nach Anspruch 4 für die Herstellung von Material, das zur pathologischen Beurteilung des myelodysplastischen Syndroms (MDS) oder von Leukämie verwendet wird.

19. Arzneimittel umfassend das Nucleinsäuremolekül nach Anspruch 1 und/oder das Nucleinsäuremolekül nach Anspruch 2 und/oder das Nucleinsäuremolekül nach Anspruch 3 und/oder das Polypeptid nach Anspruch 4 und/oder den Antikörper nach Anspruch 12 und, gegebenenfalls, einen pharmazeutisch verträglichen Träger und/oder Verdünnungsmittel.

20. Kit zum Nachweis des Polypeptids nach Anspruch 4, wobei der Kit den Antikörper nach Anspruch 6 umfasst.

## Revendications

1. Molécule d'acide nucléique d'un mutant par duplication en tandem codant la tyrosine kinase analogue à FMS 3 (FLT3), où ladite molécule d'acide nucléique a une séquence nucléotidique correspondant à:
(a) une mutation par duplication en tandem dans la séquence d'aminoacides de la juxtamembrane de FLT3, ou
(b) une mutation par duplication en tandem dans la séquence nucléotidique d'une région comprenant l'exon 11 ou les exons 11 à 12 de FLT3.

2. Molécule d'acide nucléique ayant une séquence nucléotidique correspondant à :
(a) une mutation par duplication en tandem dans la séquence d'aminoacides de la juxtamembrane de FLT3, ou
(b) une mutation par duplication en tandem dans la séquence nucléotidique d'une région comprenant l'exon 11 ou les exons 11 à 12 de FLT3.

3. Molécule d'acide nucléique capable de s'hybrider spécifiquement dans des conditions stringentes à une molécule d'acide nucléique correspondant à :
(a) une mutation par duplication en tandem dans la séquence d'aminoacides de la juxtamembrane de FLT3, ou
(b) une mutation par duplication en tandem dans la séquence nucléotidique d'une région comprenant l'exon 11 ou les exons 11 à 12 de FLT3 dans l'acide nucléique selon la revendication 1.

4. Polypeptide codé par la molécule d'acide nucléique selon la revendication 1, ou la molécule d'acide nucléique selon la revendication 2.

5. Cellule exprimant la molécule d'acide nucléique selon la revendication 1, ou la molécule d'acide nucléique selon la revendication 2, ou le polypeptide selon la revendication 4.

6. Anticorps qui est spécifique pour le polypeptide selon la revendication 4.

7. Procédé pour détecter la molécule d'acide nucléique selon la revendication 1, ou la molécule d'acide nucléique selon la revendication 2, comprenant les étapes de :
(a) soumission d'un échantillon d'acide nucléique provenant d'un humain à une réaction d'amplification génique, où un fragment d'acide nucléique comprenant l'exon 11 ou les exons 11 à 12 du gène de la tyrosine kinase analogue à FMS 3 (FLT3) et ayant une mutation par duplication en tandem dans la juxtamembrane est amplifié, qui peut être trouvé dans le gène FLT3 ;
(b) détection de la présence de la mutation par duplication en tandem dans le fragment d'acide nucléique de l'étape (a).

8. Procédé selon la revendication 7, où l'étape (b) est accomplie par comparaison du fragment d'acide nucléique amplifié obtenu dans l'étape (a) avec une séquence dérivée d'un FLT3 normal, pour détecter ainsi la présence d'une mutation par duplication en tandem dans la juxtamembrane.

9. Procédé selon la revendication 7 ou 8 où, dans l'étape (b), une mutation par longueur est utilisée comme indice de la mutation par duplication en tandem.

10. Procédé selon l'une quelconque des revendications 7 à 9, où la réaction d'amplification génique dans l'étape (a) est réalisée avec une paire d'amorces choisie dans le groupe consistant en : SEQ ID NO : 26 et 27, SEQ ID NO : 30 et 31, et SEQ ID NO : 32 et 33.

11. Procédé selon l'une quelconque des revendications 7 à 10, où la mutation par duplication en tandem n'est pas trouvée dans un gène d'un individu normal.

12. Kit pour le procédé selon l'une quelconque des revendications 7 à 11, où ledit kit comprend des amorces pour amplifier une région comprenant l'exon 11 ou les exons 11 à 12 du gène FLT3.

13. Kit selon la revendication 12 où lesdites amorces sont choisies dans le groupe consistant en : SEQ ID NO : 26 et 27, SEQ ID NO : 30 et 31, et SEQ ID NO : 32 et 33.

14. Utilisation d'une cellule souche hématopoïétique exprimant une molécule d'acide nucléique codant un polypeptide mutant par duplication en tandem et d'une cellule exprimant le FLT3 normal pour cribler un médicament pour l'examen et le traitement d'une maladie des cellules sanguines ou d'une maladie des cellules souches hématopoïétiques.

15. Utilisation selon la revendication 14 où ladite molécule d'acide nucléique a une mutation par duplication en tandem dans une séquence nucléotidique d'une juxtamembrane.

16. Utilisation d'une cellule souche hématopoïétique exprimant la molécule d'acide nucléique selon la revendication 1, ou la molécule d'acide nucléique selon la revendication 2, ou le polypeptide selon la revendication 4 et d'une cellule exprimant le FLT3 normal pour cribler un médicament pour l'examen et le traitement d'une maladie des cellules sanguines ou d'une maladie des cellules souches hématopoïétiques.

17. Utilisation de la molécule d'acide nucléique selon la revendication 1, ou de la molécule d'acide nucléique selon la revendication 2 pour la préparation d'un médicament pour réguler la prolifération, la réponse immunitaire ou la transmission d'informations de signaux d'une cellule sanguine ou d'une cellule souche hématopoïétique.

18. Utilisation de la molécule d'acide nucléique selon la revendication 1, ou de la molécule d'acide nucléique selon la revendication 2, ou du polypeptide selon la revendication 4, pour la préparation d'un produit utilisé pour le jugement pathologique du syndrome myélodysplasique (MDS) ou de la leucémie.

19. Composition pharmaceutique comprenant la molécule d'acide nucléique selon la revendication 1, et/ou la molécule d'acide nucléique selon la revendication 2, et/ou la molécule d'acide nucléique selon la revendication 3, et/ou le polypeptide selon la revendication 4, et/ou l'anticorps selon la revendication 12 et, éventuellement, un support et/ou diluant pharmaceutiquement acceptable.

20. Kit pour la détection du polypeptide selon la revendication 4, où le kit comprend l'anticorps selon la revendication 6.


### SEQUENCE LISTING

(1) GENERAL INFORMATION:
   (i) APPLICANT:
      (A) NAME: Takara Shuzo Co., Ltd.
      (B) STREET: 609, Takenaka-cho, Fushimi-ku, Kyoto-shi
      (C) CITY: Kyoto
      (E) COUNTRY: Japan
      (F) POSTAL CODE (ZIP): 612
   (ii) TITLE OF INVENTION: Nucleic acid encoding receptor type protein kinase
   (iii) NUMBER OF SEQUENCES: 33
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 97943188.9
(2) INFORMATION FOR SEQ ID NO: 1:
   (i) SEQUENCE CHARACTERISTICS:
      (A) LENGTH: 97 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 1:
   Gln Phe Arg Tyr Glu Ser Gln Leu Gln Met Val Gln Val Thr Gly Ser
   1               5                   10                  15
   Ser Asp Asn Glu Tyr Phe Tyr Val Glu Ser Gln Leu Gln Met Val Gln
               20                  25                  30
   Val Thr Gly Ser Ser Asp Ser Glu Tyr Phe Tyr Val Asp Phe Arg Glu
           35                  40                  45
   Tyr Glu Tyr Asp Leu Lys Trp Glu Phe Pro Arg Glu Asn Leu Glu Phe
       50                  55                  60
   Gly Lys Val Leu Gly Ser Gly Ala Phe Gly Lys Val Met Asn Ala Thr
   65                  70                  75                  80
   Ala Leu Glu Leu Ala Lys Gln Glu Ser Gln Ser Arg Leu Pro Ser Lys
                       85                  90                  95
   Cys
(2) INFORMATION FOR SEQ ID NO: 2:
   (i) SEQUENCE CHARACTERISTICS:
      (A) LENGTH: 100 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 2:
   Gln Phe Arg Tyr Glu Ser Gln Leu Gln Met Val Gln Val Thr Gly Ser
   1               5                   10                  15
   Ser Asp Asn Glu Tyr Phe Tyr Val Asp Phe Arg Glu Tyr Glu Tyr Asp
               20                  25                  30
   Leu Lys Trp Glu Phe Pro Arg Glu Asn Leu Glu Phe Gly Lys Val Leu
           35                  40                  45
   Gly Ser Glu Tyr Asp Leu Lys Trp Glu Phe Pro Arg Glu Asn Leu Glu
       50                  55                  60
   Phe Gly Lys Val Leu Gly Ser Gly Ala Phe Gly Lys Val Met Asn Ala
   65                  70                  75                  80
   Thr Ala Tyr Gly Ile Ser Lys Thr Gly Val Ser Ile Gln Val Ala Val
                   85                  90                  95
   Lys Met Leu Lys
               100
(2) INFORMATION FOR SEQ ID NO: 3:
   (i) SEQUENCE CHARACTERISTICS:
      (A) LENGTH: 92 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 3:
   Gln Phe Arg Tyr Glu Ser Gln Leu Gln Met Val Gln Val Thr Gly Ser
   1               5                   10                  15
   Ser Asp Asn Glu Tyr Phe Tyr Val Asp Phe Arg Glu Tyr Glu Tyr Asp
               20                  25                  30
   Leu Lys Trp Glu Phe Asp Phe Arg Glu Tyr Glu Tyr Asp Leu Lys Trp
           35                  40                  45
   Glu Phe Pro Arg Glu Asn Leu Glu Phe Gly Lys Val Leu Gly Ser Gly
       50                  55                  60
   Ala Phe Gly Lys Val Met Asn Ala Thr Ala Tyr Gly Ile Ser Lys Thr
   65                  70                  75                  80
   Gly Val Ser Ile Gln Val Ala Val Lys Met Leu Lys
                   85                  90
(2) INFORMATION FOR SEQ ID NO: 4:
   (i) SEQUENCE CHARACTERISTICS:
      (A) LENGTH: 89 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 4:
   Gln Phe Arg Tyr Glu Ser Gln Leu Gln Met Val Gln Val Thr Gly Ser
   1               5                   10                  15
   Ser Asp Asn Glu Tyr Phe Tyr Val Asp Phe Arg Glu Tyr Glu Tyr Asp
               20                  25                  30
   Leu Lys Trp Glu Phe Pro Arg Glu Asn Trp His Lys Trp Glu Phe Pro
           35                  40                  45
   Arg Glu Asn Leu Glu Phe Gly Lys Val Leu Gly Ser Gly Ala Phe Gly
       50                  55                  60
   Lys Val Met Asn Ala Thr Ala Tyr Gly Ile Ser Lys Thr Gly Val Ser
   65                  70                  75                  80
   Ile Gln Val Ala Val Lys Met Leu Lys
                   85
(2) INFORMATION FOR SEQ ID NO: 5:
   (i) SEQUENCE CHARACTERISTICS:
      (A) LENGTH: 92 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 5:
   Gln Phe Arg Tyr Glu Ser Gln Leu Gln Met Val Gln Val Thr Gly Ser
   1               5                   10                  15
   Ser Asp Asn Glu Tyr Phe Tyr Val Asp Phe Arg Gly Ser Ser Asp Asn
               20                  25                  30
   Glu Tyr Phe Tyr Val Asp Phe Arg Glu Tyr Glu Tyr Asp Leu Lys Trp
           35                  40                  45
   Glu Phe Pro Arg Glu Asn Leu Glu Phe Gly Lys Val Leu Gly Ser Gly
       50                  55                  60
   Ala Phe Gly Lys Val Met Asn Ala Thr Ala Tyr Gly Ile Ser Lys Thr
   65                  70                  75                      80
   Gly Val Ser Ile Gln Val Ala Val Lys Met Leu Lys
                   85                  90
(2) INFORMATION FOR SEQ ID NO: 6:
   (i) SEQUENCE CHARACTERISTICS:
      (A) LENGTH: 296 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 6:
   CAATTTAGGT ATGAAAGCCA GCTACAGATG GTACAGGTGA CCGGCTCCTC AGATAATGAG    60
   TACTTCTACG TTGAAAGCCA GCTACAGATG GTACAGGTGA CCGGCTCCTC AGATAGTGAG   120
   TACTTCTACG TTGATTTCAG AGAATATGAA TATGATCTCA AATGGGAGTT TCCAAGAGAA   180
   AATTTAGAGT TTGGGAAGGT ACTAGGATCA GGTGCTTTTG GAAAAGTGAT GAACGCAACA   240
   GCTTTGGAAT TAGCAAAACA GGAGTCTCAA TCCAGGTTGC CGTCAAAATG CTGAAA        296
(2) INFORMATION FOR SEQ ID NO: 7:
   (i) SEQUENCE CHARACTERISTICS:
      (A) LENGTH: 300 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 7:
   CAATTTAGGT ATGAAAGCCA GCTACAGATG GTACAGGTGA CCGGCTCCTC AGATAATGAG 60
   TACTTCTACG TTGATTTCAG AGAATATGAA TATGATCTCA AATGGGAGTT TCCAAGAGAA 120
   AATTTAGAGT TTGGGAAGGT ACTAGGATCC GAATATGATC TCAAATGGGA GTTTCCAAGA 180
   GAAAATTTAG AGTTTGGGAA GGTACTAGGA TCAGGTGCTT TTGGAAAAGT GATGAACGCA 240
   ACAGCTTATG GAATTAGCAA AACAGGAGTC TCAATCCAGG TTGCCGTCAA AATGCTGAAA 300
(2) INFORMATION FOR SEQ ID NO: 8:
   (i) SEQUENCE CHARACTERISTICS:
      (A) LENGTH: 276 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 8:
   CAATTTAGGT ATGAAAGCCA GCTACAGATG GTACAGGTGA CCGGCTCCTC AGATAATGAG 60
   TACTTCTACG TTGATTTCAG AGAATATGAA TATGATCTCA AATGGGAGTT TGATTTCAGA 120
   GAATATGAAT ATGATCTCAA ATGGGAGTTT CCAAGAGAAA ATTTAGAGTT TGGGAAGGTA 180
   CTAGGATCAG GTGCTTTTGG AAAAGTGATG AACGCAACAG CTTATGGAAT TAGCAAAACA 240
   GGAGTCTCAA TCCAGGTTGC CGTCAAAATG CTGAAA 276
(2) INFORMATION FOR SEQ ID NO: 9:
   (i) SEQUENCE CHARACTERISTICS:
      (A) LENGTH: 267 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 9:
   CAATTTAGGT ATGAAAGCCA GCTACAGATG GTACAGGTGA CCGGCTCCTC AGATAATGAG 60
   TACTTCTACG TTGATTTCAG AGAATATGAA TATGATCTCA AATGGGAGTT TCCAAGAGAA 120
   AATTGGCACA AATGGGAGTT TCCAAGAGAA AATTTAGAGT TTGGGAAGGT ACTAGGATCA 180
   GGTGCTTTTG GAAAAGTGAT GAACGCAACA GCTTATGGAA TTAGCAAAAC AGGAGTCTCA 240
   ATCCAGGTTG CCGTCAAAAT GCTGAAA 267
(2) INFORMATION FOR SEQ ID NO: 10:
   (i) SEQUENCE CHARACTERISTICS:
      (A) LENGTH: 276 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 10:
   CAATTTAGGT ATGAAAGCCA GCTACAGATG GTACAGGTGA CCGGCTCCTC AGATAATGAG 60
   TACTTCTACG TTGATTTCAG AGGCTCCTCA GATAATGAGT ACTTCTACGT TGATTTCAGA 120
   GAATATGAAT ATGATCTCAA ATGGGAGTTT CCAAGAGAAA ATTTAGAGTT TGGGAAGGTA 180
   CTAGGATCAG GTGCTTTTGG AAAAGTGATG AACGCAACAG CTTATGGAAT TAGCAAAACA 240
   GGAGTCTCAA TCCAGGTTGC CGTCAAAATG CTGAAA 276
(2) INFORMATION FOR SEQ ID NO: 11:
   (i) SEQUENCE CHARACTERISTICS:
      (A) LENGTH: 386 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 11:
   CAATTTAGGT ATGAAAGCCA GCTACAGATG GTACAGGTGA CCGGCTCCTC AGATAATGAG    60
   TACTTCTACG TTGAAAGCCA GCTACAGATG GTACAGGTGA CCGGCTCCTC AGATAGTGAG   120
   TACTTCTACG TTGATTTCAG AGAATATGAA TATGATCTCA AATGGGAGTT TCCAAGAGAA   180
   AATTTAGAGT TTGGTAAGAA TGGAATGTGC CAAATGTTTC TGCAGCATTT CTTTTCCATT   240
   GGAAAATCTT TAAAATGCAC GTACTCACCA TTTGTCTTTG CAGGGAAGGT ACTAGGATCA   300
   GGTGCTTTTG GAAAAGTGAT GAACGCAACA GCTTTGGAAT TAGCAAAACA GGAGTCTCAA   360
   TCCAGGTTGC CGTCAAAATG CTGAAA                                        386
(2) INFORMATION FOR SEQ ID NO: 12:
   (i) SEQUENCE CHARACTERISTICS:
      (A) LENGTH: 480 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 12:
   CAATTTAGGT ATGAAAGCCA GCTACAGATG GTACAGGTGA CCGGCTCCTC AGATAATGAG    60
   TACTTCTACG TTGATTTCAG AGAATATGAA TATGATCTCA AATGGGAGTT TCCAAGAGAA   120
   AATTTAGAGT TTGGTAAGAA TGGAATGTGC CAAATGTTTC TGCAGCATTT CTTTTCCATT   180
   GGAAAATCTT TAAAATGCAC GTACTCACCA TTTGTCTTTG CAGGGAAGGT ACTAGGATCC   240
   GAATATGATC TCAAATGGGA GTTTCCAAGA GAAAATTTAG AGTTTGGTGA GAATGGAATG   300
   TGCCAAATGT TTCTGCAGCA TTTCTTTTCC ATTGGAAAAT CTTTAAAATG CACGTACTCA   360
   CCATTTGTCT TTGCAGGGAA GGTACTAGGA TCAGGTGCTT TTGGAAAAGT GATGAACGCA   420
   ACAGCTTATG GAATTAGCAA AACAGGAGTC TCAATCCAGG TTGCCGTCAA AATGCTGAAA   480
(2) INFORMATION FOR SEQ ID NO: 13:
   (i) SEQUENCE CHARACTERISTICS:
      (A) LENGTH: 366 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 13:
   CAATTTAGGT ATGAAAGCCA GCTACAGATG GTACAGGTGA CCGGCTCCTC AGATAATGAG    60
   TACTTCTACG TTGATTTCAG AGAATATGAA TATGATCTCA AATGGGAGTT TGATTTCAGA   120
   GAATATGAAT ATGATCTCAA ATGGGAGTTT CCAAGAGAAA ATTTAGAGTT TGGTAAGAAT   180
   GGAATGTGCC AAATGTTTCT GCAGCATTTC TTTTCCATTG GAAAATCTTT AAAATGCACG   240
   TACTCACCAT TTGTCTTTGC AGGGAAGGTA CTAGGATCAG GTGCTTTTGG AAAAGTGATG   300
   AACGCAACAG CTTATGGAAT TAGCAAAACA GGAGTCTCAA TCCAGGTTGC CGTCAAAATG   360
   CTGAAA                                                              366
(2) INFORMATION FOR SEQ ID NO: 14:
   (i) SEQUENCE CHARACTERISTICS:
      (A) LENGTH: 357 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 14:
   CAATTTAGGT ATGAAAGCCA GCTACAGATG GTACAGGTGA CCGGCTCCTC AGATAATGAG    60
   TACTTCTACG TTGATTTCAG AGAATATGAA TATGATCTCA AATGGGAGTT TCCAAGAGAA   120
   AATTGGCACA AATGGGAGTT TCCAAGAGAA AATTTAGAGT TTGGTAAGAA TGGAATGTGC   180
   CAAACGTTTC TGCAGCATTT CTTTTCCATT GGAAAATCTT TAAAATGCAC GTACTCACCA   240
   TTTGTCTTTG CAGGGAAGGT ACTAGGATCA GGTGCTTTTG GAAAAGTGAT GAACGCAACA   300
   GCTTATGGAA TTAGCAAAAC AGGAGTCTCA ATCCAGGTTG CCGTCAAAAT GCTGAAA      357
(2) INFORMATION FOR SEQ ID NO: 15:
   (i) SEQUENCE CHARACTERISTICS:
      (A) LENGTH: 366 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 15:
   CAATTTAGGT ATGAAAGCCA GCTACAGATG GTACAGGTGA CCGGCTCCTC AGATAATGAG    60
   TACTTCTACG TTGATTTCAG AGGCTCCTCA GATAATGAGT ACTTCTACGT TGATTTCAGA   120
   GAATATGAAT ATGATCTCAA ATGGGAGTTT CCAAGAGAAA ATTTAGAGTT TGGTAAGAAT   180
   GGAATGTGCC AAATGTTTCT GCAGCATTTC TTTTCCATTG GAAAATCTTT AAAATGCACG   240
   TACTCACCAT TTGTCTTTGC AGGGAAGGTA CTAGGATCAG GTGCTTTTGG AAAAGTGATG   300
   AACGCAACAG CTTATGGAAT TAGCAAAACA GGAGTCTCAA TCCAGGTTGC CGTCAAAATG   360
   CTGAAA                                                              366
(2) INFORMATION FOR SEQ ID NO: 16:
   (i) SEQUENCE CHARACTERISTICS:
      (A) LENGTH: 665 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 16:
   Met Pro Ala Leu Ala Arg Asp Ala Gly Thr Val Pro Leu Leu Val Val
   1               5                   10                  15
   Phe Ser Ala Met Ile Phe Gly Thr Ile Thr Asn Gln Asp Leu Pro Val
               20                  25                  30
   Ile Lys Cys Val Leu Ile Asn His Lys Asn Asn Asp Ser Ser Val Gly
           35                  40                  45
   Lys Ser Ser Ser Tyr Pro Met Val Ser Glu Ser Pro Glu Asp Leu Gly
       50                  55                  60
   Cys Ala Leu Arg Pro Gln Ser Ser Gly Thr Val Tyr Glu Ala Ala Ala
   65                  70                  75                  80
   Val Glu Val Asp Val Ser Ala Ser Ile Thr Leu Gln Val Leu Val Asp
                   85                  90                  95
   Ala Pro Gly Asn Ile Ser Cys Leu Trp Val Phe Lys His Ser Ser Leu
               100                 105                 110
   Asn Cys Gln Pro His Phe Asp Leu Gln Asn Arg Gly Val Val Ser Met
           115                 120                 125
   Val Ile Leu Lys Met Thr Glu Thr Gln Ala Gly Glu Tyr Leu Leu Phe
       130                 135                 140
   Ile Gln Ser Glu Ala Thr Asn Tyr Thr Ile Leu Phe Thr Val Ser Ile
   145                 150                 155                 160
   Arg Asn Thr Leu Leu Tyr Thr Leu Arg Arg Pro Tyr Phe Arg Lys Met
                   165                 170                 175
   Glu Asn Gln Asp Ala Leu Val Cys Ile Ser Glu Ser Val Pro Glu Pro
               180                 185                 190
   Ile Val Glu Trp Val Leu Cys Asp Ser Gln Gly Glu Ser Cys Lys Glu
           195                 200                 205
   Glu Ser Pro Ala Val Val Lys Lys Glu Glu Lys Val Leu His Glu Leu
       210                 215                 220
   Phe Gly Thr Asp Ile Arg Cys Cys Ala Arg Asn Glu Leu Gly Arg Glu
   225                 230                 235                 240
   Cys Thr Arg Leu Phe Thr Ile Asp Leu Asn Gln Thr Pro Gln Thr Thr
                   245                 250                 255
   Leu Pro Gln Leu Phe Leu Lys Val Gly Glu Pro Leu Trp Ile Arg Cys
               260                 265                 270
   Lys Ala Val His Val Asn His Gly Phe Gly Leu Thr Trp Glu Leu Glu
           275                 280                 285
   Asn Lys Ala Leu Glu Glu Gly Asn Tyr Phe Glu Met Ser Thr Tyr Ser
       290                 295                 300
   Thr Asn Arg Thr Met Ile Arg Ile Leu Phe Ala Phe Val Ser Ser Val
   305                 310                 315                 320
   Ala Arg Asn Asp Thr Gly Tyr Tyr Thr Cys Ser Ser Ser Lys His Pro
                   325                 330                 335
   Ser Gln Ser Ala Leu Val Thr Ile Val Gly Lys Gly Phe Ile Asn Ala
               340                 345                 350
   Thr Asn Ser Ser Glu Asp Tyr Glu Ile Asp Gln Tyr Glu Glu Phe Cys
           355                 360                 365
   Phe Ser Val Arg Phe Lys Ala Tyr Pro Gln Ile Arg Cys Thr Trp Thr
       370                 375                 380
   Phe Ser Arg Lys Ser Phe Pro Cys Glu Gln Lys Gly Leu Asp Asn Gly
   385                 390                 395                 400
   Tyr Ser Ile Ser Lys Phe Cys Asn His Lys His Gln Pro Gly Glu Tyr
                   405                 410                 415
   Ile Phe His Ala Glu Asn Asp Asp Ala Gln Phe Thr Lys Met Phe Thr
               420                 425                 430
   Leu Asn Ile Arg Arg Lys Pro Gln Val Leu Ala Glu Ala Ser Ala Ser
           435                     440                     445
   Gln Ala Ser Cys Phe Ser Asp Gly Tyr Pro Leu Pro Ser Trp Thr Trp
       450                     455                     460
   Lys Lys Cys Ser Asp Lys Ser Pro Asn Cys Thr Glu Glu Ile Thr Glu
   465                     470                     475                     480
   Gly Val Trp Asn Arg Lys Ala Asn Arg Lys Val Phe Gly Gln Trp Val
                   485                     490                     495
   Ser Ser Ser Thr Leu Asn Met Ser Glu Ala Ile Lys Gly Phe Leu Val
               500                     505                     510
   Lys Cys Cys Ala Tyr Asn Ser Leu Gly Thr Ser Cys Glu Thr Ile Leu
           515                     520                     525
   Leu Asn Ser Pro Gly Pro Phe Pro Phe Ile Gln Asp Asn Ile Ser Phe
       530                     535                     540
   Tyr Ala Thr Ile Gly Val Cys Leu Leu Phe Ile Val Val Leu Thr Leu
   545                     550                     555                     560
   Leu Ile Cys His Lys Tyr Lys Lys Gln Phe Arg Tyr Glu Ser Gln Leu
                   565                     570                     575
   Gln Met Val Gln Val Thr Gly Ser Ser Asp Asn Glu Tyr Phe Tyr Val
               580                     585                     590
   Glu Ser Gln Leu Gln Met Val Gln Val Thr Gly Ser Ser Asp Ser Glu
           595                     600                     605
   Tyr Phe Tyr Val Asp Phe Arg Glu Tyr Glu Tyr Asp Leu Lys Trp Glu
       610                     615                     620
   Phe Pro Arg Glu Asn Leu Glu Phe Gly Lys Val Leu Gly Ser Gly Ala
   625                     630                     635                     640
   Phe Gly Lys Val Met Asn Ala Thr Ala Leu Glu Leu Ala Lys Gln Glu
                   645                     650                     655
   Ser Gln Ser Arg Leu Pro Ser Lys Cys
               660                     665
(2) INFORMATION FOR SEQ ID NO: 17:
   (i) SEQUENCE CHARACTERISTICS:
      (A) LENGTH: 994 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 17:
   Met Pro Ala Leu Ala Arg Asp Ala Gly Thr Val Pro Leu Leu Val Val
   1               5                   10                  15
   Phe Ser Ala Met Ile Phe Gly Thr Ile Thr Asn Gln Asp Leu Pro Val
               20                  25                  30
   Ile Lys Cys Val Leu Ile Asn His Lys Asn Asn Asp Ser Ser Val Gly
           35                  40                  45
   Lys Ser Ser Ser Tyr Pro Met Val Ser Glu Ser Pro Glu Asp Leu Gly
       50                  55                  60
   Cys Ala Leu Arg Pro Gln Ser Ser Gly Thr Val Tyr Glu Ala Ala Ala
   65                  70                  75                  80
   Val Glu Val Asp Val Ser Ala Ser Ile Thr Leu Gln Val Leu Val Asp
                   85                  90                  95
   Ala Pro Gly Asn Ile Ser Cys Leu Trp Val Phe Lys His Ser Ser Leu
               100                 105                 110
   Asn Cys Gln Pro His Phe Asp Leu Gln Asn Arg Gly Val Val Ser Met
           115                 120                 125
   Val Ile Leu Lys Met Thr Glu Thr Gln Ala Gly Glu Tyr Leu Leu Phe
       130                 135                 140
   Ile Gln Ser Glu Ala Thr Asn Tyr Thr Ile Leu Phe Thr Val Ser Ile
   145                 150                 155                 160
   Arg Asn Thr Leu Leu Tyr Thr Leu Arg Arg Pro Tyr Phe Arg Lys Met
                   165                 170                 175
   Glu Asn Gln Asp Ala Leu Val Cys Ile Ser Glu Ser Val Pro Glu Pro
               180                 185                 190
   Ile Val Glu Trp Val Leu Cys Asp Ser Gln Gly Glu Ser Cys Lys Glu
           195                 200                 205
   Glu Ser Pro Ala Val Val Lys Lys Glu Glu Lys Val Leu His Glu Leu
       210                 215                 220
   Phe Gly Thr Asp Ile Arg Cys Cys Ala Arg Asn Glu Leu Gly Arg Glu
   225                 230                 235                 240
   Cys Thr Arg Leu Phe Thr Ile Asp Leu Asn Gln Thr Pro Gln Thr Thr
                   245                 250                 255
   Leu Pro Gln Leu Phe Leu Lys Val Gly Glu Pro Leu Trp Ile Arg Cys
               260                 265                 270
   Lys Ala Val His Val Asn His Gly Phe Gly Leu Thr Trp Glu Leu Glu
           275                 280                 285
   Asn Lys Ala Leu Glu Glu Gly Asn Tyr Phe Glu Met Ser Thr Tyr Ser
       290                 295                 300
   Thr Asn Arg Thr Met Ile Arg Ile Leu Phe Ala Phe Val Ser Ser Val
   305                 310                 315                 320
   Ala Arg Asn Asp Thr Gly Tyr Tyr Thr Cys Ser Ser Ser Lys His Pro
                   325                 330                 335
   Ser Gln Ser Ala Leu Val Thr Ile Val Gly Lys Gly Phe Ile Asn Ala
               340                 345                 350
   Thr Asn Ser Ser Glu Asp Tyr Glu Ile Asp Gln Tyr Glu Glu Phe Cys
           355                 360                 365
   Phe Ser Val Arg Phe Lys Ala Tyr Pro Gln Ile Arg Cys Thr Trp Thr
       370                 375                 380
   Phe Ser Arg Lys Ser Phe Pro Cys Glu Gln Lys Gly Leu Asp Asn Gly
   385                 390                 395                 400
   Tyr Ser Ile Ser Lys Phe Cys Asn His Lys His Gln Pro Gly Glu Tyr
                   405                 410                 415
   Ile Phe His Ala Glu Asn Asp Asp Ala Gln Phe Thr Lys Met Phe Thr
               420                 425                 430
   Leu Asn Ile Arg Arg Lys Pro Gln Val Leu Ala Glu Ala Ser Ala Ser
           435                 440                 445
   Gln Ala Ser Cys Phe Ser Asp Gly Tyr Pro Leu Pro Ser Trp Thr Trp
       450                 455                 460
   Lys Lys Cys Ser Asp Lys Ser Pro Asn Cys Thr Glu Glu Ile Thr Glu
   465                 470                 475                 480
   Gly Val Trp Asn Arg Lys Ala Asn Arg Lys Val Phe Gly Gln Trp Val
                   485                 490                 495
   Ser Ser Ser Thr Leu Asn Met Ser Glu Ala Ile Lys Gly Phe Leu Val
               500                 505                 510
   Lys Cys Cys Ala Tyr Asn Ser Leu Gly Thr Ser Cys Glu Thr Ile Leu
           515                 520                 525
   Leu Asn Ser Pro Gly Pro Phe Pro Phe Ile Gln Asp Asn Ile Ser Phe
       530                 535                 540
   Tyr Ala Thr Ile Gly Val Cys Leu Leu Phe Ile Val Val Leu Thr Leu
   545                 550                 555                 560
   Leu Ile Cys His Lys Tyr Lys Lys Gln Phe Arg Tyr Glu Ser Gln Leu
                   565                 570                 575
   Gln Met Val Gln Val Thr Gly Ser Ser Asp Asn Glu Tyr Phe Tyr Val
               580                 585                 590
   Asp Phe Arg Glu Tyr Glu Tyr Asp Leu Lys Trp Glu Phe Pro Arg Glu
   595 600 605
   Asn Leu Glu Phe Gly Lys Val Leu Gly Ser Glu Tyr Asp Leu Lys Trp
   610 615 620
   Glu Phe Pro Arg Glu Asn Leu Glu Phe Gly Lys Val Leu Gly Ser Gly
   625 630 635 640
   Ala Phe Gly Lys Val Met Asn Ala Thr Ala Tyr Gly Ile Ser Lys Thr
   645 650 655
   Gly Val Ser Ile Gln Val Ala Val Lys Met Leu Lys Glu Lys Ala Asp
   660 665 670
   Ser Ser Glu Arg Glu Ala Leu Met Ser Glu Leu Lys Met Met Thr Gln
   675 680 685
   Leu Gly Ser His Glu Asn Ile Val Asn Leu Leu Gly Ala Cys Thr Leu
   690 695 700
   Ser Gly Pro Ile Tyr Leu Ile Phe Glu Tyr Cys Cys Tyr Gly Asp Leu
   705 710 715 720
   Leu Asn Tyr Leu Arg Ser Lys Arg Glu Lys Phe His Arg Thr Trp Thr
   725 730 735
   Glu Ile Phe Lys Glu His Asn Phe Ser Phe Tyr Pro Thr Phe Gln Ser
   740 745 750
   His Pro Asn Ser Ser Met Pro Gly Ser Arg Glu Val Gln Ile His Pro
   755 760 765
   Asp Ser Asp Gln Ile Ser Gly Leu His Gly Asn Ser Phe His Ser Glu
   770 775 780
   Asp Glu Ile Glu Tyr Glu Asn Gln Lys Arg Leu Glu Glu Glu Glu Asp
   785 790 795 800
   Leu Asn Val Leu Thr Phe Glu Asp Leu Leu Cys Phe Ala Tyr Gln Val
   805 810 815
   Ala Lys Gly Met Glu Phe Leu Glu Phe Lys Ser Cys Val His Arg Asp
   820 825 830
   Leu Ala Ala Arg Asn Val Leu Val Thr His Gly Lys Val Val Lys Ile
   835 840 845
   Cys Asp Phe Gly Leu Ala Arg Asp Ile Met Ser Asp Ser Asn Tyr Val
   850 855 860
   Val Arg Gly Asn Ala Arg Leu Pro Val Lys Trp Met Ala Pro Glu Ser
   865 870 875 880
   Leu Phe Glu Gly Ile Tyr Thr Ile Lys Ser Asp Val Trp Ser Tyr Gly
   885 890 895
   Ile Leu Leu Trp Glu Ile Phe Ser Leu Gly Val Asn Pro Tyr Pro Gly
   900 905 910
   Ile Pro Val Asp Ala Asn Phe Tyr Lys Leu Ile Gln Asn Gly Phe Lys
           915                 920                 925
   Met Asp Gln Pro Phe Tyr Ala Thr Glu Glu Ile Tyr Ile Ile Met Gln
       930                 935                 940
   Ser Cys Trp Ala Phe Asp Ser Arg Lys Arg Pro Ser Phe Pro Asn Leu
   945                 950                 955                 960
   Thr Ser Phe Leu Gly Cys Gln Leu Ala Asp Ala Glu Glu Ala Met Tyr
                   965                 970                 975
   Gln Asn Val Asp Gly Arg Val Ser Glu Cys Pro His Thr Tyr Gln Asn
               980                 985                 990
   Arg Arg
(2) INFORMATION FOR SEQ ID NO: 18:
   (i) SEQUENCE CHARACTERISTICS:
      (A) LENGTH: 986 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 18:
   Met Pro Ala Leu Ala Arg Asp Ala Gly Thr Val Pro Leu Leu Val Val
   1               5                   10                  15
   Phe Ser Ala Met Ile Phe Gly Thr Ile Thr Asn Gln Asp Leu Pro Val
               20                  25                  30
   Ile Lys Cys Val Leu Ile Asn His Lys Asn Asn Asp Ser Ser Val Gly
           35                  40                  45
   Lys Ser Ser Ser Tyr Pro Met Val Ser Glu Ser Pro Glu Asp Leu Gly
       50                  55                  60
   Cys Ala Leu Arg Pro Gln Ser Ser Gly Thr Val Tyr Glu Ala Ala Ala
   65                  70                  75                  80
   Val Glu Val Asp Val Ser Ala Ser Ile Thr Leu Gln Val Leu Val Asp
                   85                  90                  95
   Ala Pro Gly Asn Ile Ser Cys Leu Trp Val Phe Lys His Ser Ser Leu
               100                 105                 110
   Asn Cys Gln Pro His Phe Asp Leu Gln Asn Arg Gly Val Val Ser Met
           115                 120                 125
   Val Ile Leu Lys Met Thr Glu Thr Gln Ala Gly Glu Tyr Leu Leu Phe
       130                 135                 140
   Ile Gln Ser Glu Ala Thr Asn Tyr Thr Ile Leu Phe Thr Val Ser Ile
   145                 150                 155                 160
   Arg Asn Thr Leu Leu Tyr Thr Leu Arg Arg Pro Tyr Phe Arg Lys Met
                   165                 170                 175
   Glu Asn Gln Asp Ala Leu Val Cys Ile Ser Glu Ser Val Pro Glu Pro
               180                 185                 190
   Ile Val Glu Trp Val Leu Cys Asp Ser Gln Gly Glu Ser Cys Lys Glu
           195                 200                 205
   Glu Ser Pro Ala Val Val Lys Lys Glu Glu Lys Val Leu His Glu Leu
       210                 215                 220
   Phe Gly Thr Asp Ile Arg Cys Cys Ala Arg Asn Glu Leu Gly Arg Glu
   225                 230                 235                 240
   Cys Thr Arg Leu Phe Thr Ile Asp Leu Asn Gln Thr Pro Gln Thr Thr
                   245                 250                 255
   Leu Pro Gln Leu Phe Leu Lys Val Gly Glu Pro Leu Trp Ile Arg Cys
               260                 265                 270
   Lys Ala Val His Val Asn His Gly Phe Gly Leu Thr Trp Glu Leu Glu
           275                 280                 285
   Asn Lys Ala Leu Glu Glu Gly Asn Tyr Phe Glu Met Ser Thr Tyr Ser
       290                 295                 300
   Thr Asn Arg Thr Met Ile Arg Ile Leu Phe Ala Phe Val Ser Ser Val
   305                 310                 315                 320
   Ala Arg Asn Asp Thr Gly Tyr Tyr Thr Cys Ser Ser Ser Lys His Pro
                   325                 330                 335
   Ser Gln Ser Ala Leu Val Thr Ile Val Gly Lys Gly Phe Ile Asn Ala
               340                 345                 350
   Thr Asn Ser Ser Glu Asp Tyr Glu Ile Asp Gln Tyr Glu Glu Phe Cys
           355                 360                 365
   Phe Ser Val Arg Phe Lys Ala Tyr Pro Gln Ile Arg Cys Thr Trp Thr
       370                 375                 380
   Phe Ser Arg Lys Ser Phe Pro Cys Glu Gln Lys Gly Leu Asp Asn Gly
   385                 390                 395                 400
   Tyr Ser Ile Ser Lys Phe Cys Asn His Lys His Gln Pro Gly Glu Tyr
                   405                 410                 415
   Ile Phe His Ala Glu Asn Asp Asp Ala Gln Phe Thr Lys Met Phe Thr
               420                 425                 430
   Leu Asn Ile Arg Arg Lys Pro Gln Val Leu Ala Glu Ala Ser Ala Ser
           435                 440                 445
   Gln Ala Ser Cys Phe Ser Asp Gly Tyr Pro Leu Pro Ser Trp Thr Trp
       450                 455                 460
   Lys Lys Cys Ser Asp Lys Ser Pro Asn Cys Thr Glu Glu Ile Thr Glu
   465                 470                 475                 480
   Gly Val Trp Asn Arg Lys Ala Asn Arg Lys Val Phe Gly Gln Trp Val
                   485                 490                 495
   Ser Ser Ser Thr Leu Asn Met Ser Glu Ala Ile Lys Gly Phe Leu Val
               500                 505                 510
   Lys Cys Cys Ala Tyr Asn Ser Leu Gly Thr Ser Cys Glu Thr Ile Leu
           515                 520                 525
   Leu Asn Ser Pro Gly Pro Phe Pro Phe Ile Gln Asp Asn Ile Ser Phe
       530                 535                 540
   Tyr Ala Thr Ile Gly Val Cys Leu Leu Phe Ile Val Val Leu Thr Leu
   545                 550                 555                 560
   Leu Ile Cys His Lys Tyr Lys Lys Gln Phe Arg Tyr Glu Ser Gln Leu
                   565                 570                 575
   Gln Met Val Gln Val Thr Gly Ser Ser Asp Asn Glu Tyr Phe Tyr Val
               580                 585                 590
   Asp Phe Arg Glu Tyr Glu Tyr Asp Leu Lys Trp Glu Phe Asp Phe Arg
           595                 600                 605
   Glu Tyr Glu Tyr Asp Leu Lys Trp Glu Phe Pro Arg Glu Asn Leu Glu
       610                 615                 620
   Phe Gly Lys Val Leu Gly Ser Gly Ala Phe Gly Lys Val Met Asn Ala
   625                 630                 635                 640
   Thr Ala Tyr Gly Ile Ser Lys Thr Gly Val Ser Ile Gln Val Ala Val
                   645                 650                 655
   Lys Met Leu Lys Glu Lys Ala Asp Ser Ser Glu Arg Glu Ala Leu Met
               660                 665                 670
   Ser Glu Leu Lys Met Met Thr Gln Leu Gly Ser His Glu Asn Ile Val
           675                 680                 685
   Asn Leu Leu Gly Ala Cys Thr Leu Ser Gly Pro Ile Tyr Leu Ile Phe
       690                 695                 700
   Glu Tyr Cys Cys Tyr Gly Asp Leu Leu Asn Tyr Leu Arg Ser Lys Arg
   705                 710                 715                 720
   Glu Lys Phe His Arg Thr Trp Thr Glu Ile Phe Lys Glu His Asn Phe
                   725                 730                 735
   Ser Phe Tyr Pro Thr Phe Gln Ser His Pro Asn Ser Ser Met Pro Gly
                   740                 745                 750
   Ser Arg Glu Val Gln Ile His Pro Asp Ser Asp Gln Ile Ser Gly Leu
               755                 760                 765
   His Gly Asn Ser Phe His Ser Glu Asp Glu Ile Glu Tyr Glu Asn Gln
           770                 775                 780
   Lys Arg Leu Glu Glu Glu Glu Asp Leu Asn Val Leu Thr Phe Glu Asp
   785                 790                 795                 800
   Leu Leu Cys Phe Ala Tyr Gln Val Ala Lys Gly Met Glu Phe Leu Glu
                   805                 810                 815
   Phe Lys Ser Cys Val His Arg Asp Leu Ala Ala Arg Asn Val Leu Val
               820                 825                 830
   Thr His Gly Lys Val Val Lys Ile Cys Asp Phe Gly Leu Ala Arg Asp
           835                 840                 845
   Ile Met Ser Asp Ser Asn Tyr Val Val Arg Gly Asn Ala Arg Leu Pro
       850                 855                 860
   Val Lys Trp Met Ala Pro Glu Ser Leu Phe Glu Gly Ile Tyr Thr Ile
   865                 870                 875                 880
   Lys Ser Asp Val Trp Ser Tyr Gly Ile Leu Leu Trp Glu Ile Phe Ser
                   885                 890                 895
   Leu Gly Val Asn Pro Tyr Pro Gly Ile Pro Val Asp Ala Asn Phe Tyr
               900                 905                 910
   Lys Leu Ile Gln Asn Gly Phe Lys Met Asp Gln Pro Phe Tyr Ala Thr
           915                 920                 925
   Glu Glu Ile Tyr Ile Ile Met Gln Ser Cys Trp Ala Phe Asp Ser Arg
       930                 935                 940
   Lys Arg Pro Ser Phe Pro Asn Leu Thr Ser Phe Leu Gly Cys Gln Leu
   945                 950                 955                 960
   Ala Asp Ala Glu Glu Ala Met Tyr Gln Asn Val Asp Gly Arg Val Ser
                   965                 970                 975
   Glu Cys Pro His Thr Tyr Gln Asn Arg Arg
               980                 985
(2) INFORMATION FOR SEQ ID NO: 19:
   (i) SEQUENCE CHARACTERISTICS:
      (A) LENGTH: 983 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 19:
   Met Pro Ala Leu Ala Arg Asp Ala Gly Thr Val Pro Leu Leu Val Val
   1               5                   10                  15
   Phe Ser Ala Met Ile Phe Gly Thr Ile Thr Asn Gln Asp Leu Pro Val
               20                  25                  30
   Ile Lys Cys Val Leu Ile Asn His Lys Asn Asn Asp Ser Ser Val Gly
           35                  40                  45
   Lys Ser Ser Ser Tyr Pro Met Val Ser Glu Ser Pro Glu Asp Leu Gly
       50                  55                  60
   Cys Ala Leu Arg Pro Gln Ser Ser Gly Thr Val Tyr Glu Ala Ala Ala
   65                  70                  75                  80
   Val Glu Val Asp Val Ser Ala Ser Ile Thr Leu Gln Val Leu Val Asp
                   85                  90                  95
   Ala Pro Gly Asn Ile Ser Cys Leu Trp Val Phe Lys His Ser Ser Leu
               100                 105                 110
   Asn Cys Gln Pro His Phe Asp Leu Gln Asn Arg Gly Val Val Ser Met
           115                 120                 125
   Val Ile Leu Lys Met Thr Glu Thr Gln Ala Gly Glu Tyr Leu Leu Phe
       130                 135                 140
   Ile Gln Ser Glu Ala Thr Asn Tyr Thr Ile Leu Phe Thr Val Ser Ile
   145                 150                 155                 160
   Arg Asn Thr Leu Leu Tyr Thr Leu Arg Arg Pro Tyr Phe Arg Lys Met
                   165                 170                 175
   Glu Asn Gln Asp Ala Leu Val Cys Ile Ser Glu Ser Val Pro Glu Pro
               180                 185                 190
   Ile Val Glu Trp Val Leu Cys Asp Ser Gln Gly Glu Ser Cys Lys Glu
           195                 200                 205
   Glu Ser Pro Ala Val Val Lys Lys Glu Glu Lys Val Leu His Glu Leu
       210                 215                 220
   Phe Gly Thr Asp Ile Arg Cys Cys Ala Arg Asn Glu Leu Gly Arg Glu
   225                 230                 235                 240
   Cys Thr Arg Leu Phe Thr Ile Asp Leu Asn Gln Thr Pro Gln Thr Thr
                   245                 250                 255
   Leu Pro Gln Leu Phe Leu Lys Val Gly Glu Pro Leu Trp Ile Arg Cys
               260                 265                 270
   Lys Ala Val His Val Asn His Gly Phe Gly Leu Thr Trp Glu Leu Glu
           275                 280                 285
   Asn Lys Ala Leu Glu Glu Gly Asn Tyr Phe Glu Met Ser Thr Tyr Ser
       290                 295                 300
   Thr Asn Arg Thr Met Ile Arg Ile Leu Phe Ala Phe Val Ser Ser Val
   305                 310                 315                 320
   Ala Arg Asn Asp Thr Gly Tyr Tyr Thr Cys Ser Ser Ser Lys His Pro
                   325                 330                 335
   Ser Gln Ser Ala Leu Val Thr Ile Val Gly Lys Gly Phe Ile Asn Ala
               340                 345                 350
   Thr Asn Ser Ser Glu Asp Tyr Glu Ile Asp Gln Tyr Glu Glu Phe Cys
           355                 360                 365
   Phe Ser Val Arg Phe Lys Ala Tyr Pro Gln Ile Arg Cys Thr Trp Thr
       370                 375                 380
   Phe Ser Arg Lys Ser Phe Pro Cys Glu Gln Lys Gly Leu Asp Asn Gly
   385                 390                 395                 400
   Tyr Ser Ile Ser Lys Phe Cys Asn His Lys His Gln Pro Gly Glu Tyr
                   405                 410                 415
   Ile Phe His Ala Glu Asn Asp Asp Ala Gln Phe Thr Lys Met Phe Thr
               420                 425                 430
   Leu Asn Ile Arg Arg Lys Pro Gln Val Leu Ala Glu Ala Ser Ala Ser
           435                 440                 445
   Gln Ala Ser Cys Phe Ser Asp Gly Tyr Pro Leu Pro Ser Trp Thr Trp
       450                 455                 460
   Lys Lys Cys Ser Asp Lys Ser Pro Asn Cys Thr Glu Glu Ile Thr Glu
   465                 470                 475                 480
   Gly Val Trp Asn Arg Lys Ala Asn Arg Lys Val Phe Gly Gln Trp Val
                   485                 490                 495
   Ser Ser Ser Thr Leu Asn Met Ser Glu Ala Ile Lys Gly Phe Leu Val
               500                 505                 510
   Lys Cys Cys Ala Tyr Asn Ser Leu Gly Thr Ser Cys Glu Thr Ile Leu
           515                 520                 525
   Leu Asn Ser Pro Gly Pro Phe Pro Phe Ile Gln Asp Asn Ile Ser Phe
       530                 535                 540
   Tyr Ala Thr Ile Gly Val Cys Leu Leu Phe Ile Val Val Leu Thr Leu
   545                 550                 555                 560
   Leu Ile Cys His Lys Tyr Lys Lys Gln Phe Arg Tyr Glu Ser Gln Leu
                   565                 570                 575
   Gln Met Val Gln Val Thr Gly Ser Ser Asp Asn Glu Tyr Phe Tyr Val
               580                 585                 590
   Asp Phe Arg Glu Tyr Glu Tyr Asp Leu Lys Trp Glu Phe Pro Arg Glu
   595 600 605
   Asn Trp His Lys Trp Glu Phe Pro Arg Glu Asn Leu Glu Phe Gly Lys
   610 615 620
   Val Leu Gly Ser Gly Ala Phe Gly Lys Val Met Asn Ala Thr Ala Tyr
   625 630 635 640
   Gly Ile Ser Lys Thr Gly Val Ser Ile Gln Val Ala Val Lys Met Leu
   645 650 655
   Lys Glu Lys Ala Asp Ser Ser Glu Arg Glu Ala Leu Met Ser Glu Leu
   660 665 670
   Lys Met Met Thr Gln Leu Gly Ser His Glu Asn Ile Val Asn Leu Leu
   675 680 685
   Gly Ala Cys Thr Leu Ser Gly Pro Ile Tyr Leu Ile Phe Glu Tyr Cys
   690 695 700
   Cys Tyr Gly Asp Leu Leu Asn Tyr Leu Arg Ser Lys Arg Glu Lys Phe
   705 710 715 720
   His Arg Thr Trp Thr Glu Ile Phe Lys Glu His Asn Phe Ser Phe Tyr
   725 730 735
   Pro Thr Phe Gln Ser His Pro Asn Ser Ser Met Pro Gly Ser Arg Glu
   740 745 750
   Val Gln Ile His Pro Asp Ser Asp Gln Ile Ser Gly Leu His Gly Asn
   755 760 765
   Ser Phe His Ser Glu Asp Glu Ile Glu Tyr Glu Asn Gln Lys Arg Leu
   770 775 780
   Glu Glu Glu Glu Asp Leu Asn Val Leu Thr Phe Glu Asp Leu Leu Cys
   785 790 795 800
   Phe Ala Tyr Gln Val Ala Lys Gly Met Glu Phe Leu Glu Phe Lys Ser
   805 810 815
   Cys Val His Arg Asp Leu Ala Ala Arg Asn Val Leu Val Thr His Gly
   820 825 830
   Lys Val Val Lys Ile Cys Asp Phe Gly Leu Ala Arg Asp Ile Met Ser
   835 840 845
   Asp Ser Asn Tyr Val Val Arg Gly Asn Ala Arg Leu Pro Val Lys Trp
   850 855 860
   Met Ala Pro Glu Ser Leu Phe Glu Gly Ile Tyr Thr Ile Lys Ser Asp
   865 870 875 880
   Val Trp Ser Tyr Gly Ile Leu Leu Trp Glu Ile Phe Ser Leu Gly Val
   885 890 895
   Asn Pro Tyr Pro Gly Ile Pro Val Asp Ala Asn Phe Tyr Lys Leu Ile
                   900                     905                     910
   Gln Asn Gly Phe Lys Met Asp Gln Pro Phe Tyr Ala Thr Glu Glu Ile
               915                     920                     925
   Tyr Ile Ile Met Gln Ser Cys Trp Ala Phe Asp Ser Arg Lys Arg Pro
           930                     935                     940
   Ser Phe Pro Asn Leu Thr Ser Phe Leu Gly Cys Gln Leu Ala Asp Ala
   945                     950                     955                     960
   Glu Glu Ala Met Tyr Gln Asn Val Asp Gly Arg Val Ser Glu Cys Pro
                   965                     970                     975
   His Thr Tyr Gln Asn Arg Arg
                   980
(2) INFORMATION FOR SEQ ID NO: 20:
   (i) SEQUENCE CHARACTERISTICS:
      (A) LENGTH: 986 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 20:
   Met Pro Ala Leu Ala Arg Asp Ala Gly Thr Val Pro Leu Leu Val Val
   1               5                   10                  15
   Phe Ser Ala Met Ile Phe Gly Thr Ile Thr Asn Gln Asp Leu Pro Val
               20                  25                  30
   Ile Lys Cys Val Leu Ile Asn His Lys Asn Asn Asp Ser Ser Val Gly
           35                  40                  45
   Lys Ser Ser Ser Tyr Pro Met Val Ser Glu Ser Pro Glu Asp Leu Gly
       50                  55                  60
   Cys Ala Leu Arg Pro Gln Ser Ser Gly Thr Val Tyr Glu Ala Ala Ala
   65                  70                  75                  80
   Val Glu Val Asp Val Ser Ala Ser Ile Thr Leu Gln Val Leu Val Asp
                   85                  90                  95
   Ala Pro Gly Asn Ile Ser Cys Leu Trp Val Phe Lys His Ser Ser Leu
               100                 105                 110
   Asn Cys Gln Pro His Phe Asp Leu Gln Asn Arg Gly Val Val Ser Met
           115                 120                 125
   Val Ile Leu Lys Met Thr Glu Thr Gln Ala Gly Glu Tyr Leu Leu Phe
       130                 135                 140
   Ile Gln Ser Glu Ala Thr Asn Tyr Thr Ile Leu Phe Thr Val Ser Ile
   145                 150                 155                 160
   Arg Asn Thr Leu Leu Tyr Thr Leu Arg Arg Pro Tyr Phe Arg Lys Met
                   165                 170                 175
   Glu Asn Gln Asp Ala Leu Val Cys Ile Ser Glu Ser Val Pro Glu Pro
               180                 185                 190
   Ile Val Glu Trp Val Leu Cys Asp Ser Gln Gly Glu Ser Cys Lys Glu
           195                 200                 205
   Glu Ser Pro Ala Val Val Lys Lys Glu Glu Lys Val Leu His Glu Leu
       210                 215                 220
   Phe Gly Thr Asp Ile Arg Cys Cys Ala Arg Asn Glu Leu Gly Arg Glu
   225                 230                 235                 240
   Cys Thr Arg Leu Phe Thr Ile Asp Leu Asn Gln Thr Pro Gln Thr Thr
                   245                 250                 255
   Leu Pro Gln Leu Phe Leu Lys Val Gly Glu Pro Leu Trp Ile Arg Cys
               260                 265                 270
   Lys Ala Val His Val Asn His Gly Phe Gly Leu Thr Trp Glu Leu Glu
           275                 280                 285
   Asn Lys Ala Leu Glu Glu Gly Asn Tyr Phe Glu Met Ser Thr Tyr Ser
       290                 295                 300
   Thr Asn Arg Thr Met Ile Arg Ile Leu Phe Ala Phe Val Ser Ser Val
   305                 310                 315                 320
   Ala Arg Asn Asp Thr Gly Tyr Tyr Thr Cys Ser Ser Ser Lys His Pro
                   325                 330                 335
   Ser Gln Ser Ala Leu Val Thr Ile Val Gly Lys Gly Phe Ile Asn Ala
               340                 345                 350
   Thr Asn Ser Ser Glu Asp Tyr Glu Ile Asp Gln Tyr Glu Glu Phe Cys
           355                 360                 365
   Phe Ser Val Arg Phe Lys Ala Tyr Pro Gln Ile Arg Cys Thr Trp Thr
       370                 375                 380
   Phe Ser Arg Lys Ser Phe Pro Cys Glu Gln Lys Gly Leu Asp Asn Gly
   385                 390                 395                 400
   Tyr Ser Ile Ser Lys Phe Cys Asn His Lys His Gln Pro Gly Glu Tyr
                   405                 410                 415
   Ile Phe His Ala Glu Asn Asp Asp Ala Gln Phe Thr Lys Met Phe Thr
               420                 425                 430
   Leu Asn Ile Arg Arg Lys Pro Gln Val Leu Ala Glu Ala Ser Ala Ser
           435                 440                 445
   Gln Ala Ser Cys Phe Ser Asp Gly Tyr Pro Leu Pro Ser Trp Thr Trp
       450                 455                 460
   Lys Lys Cys Ser Asp Lys Ser Pro Asn Cys Thr Glu Glu Ile Thr Glu
   465                 470                 475                 480
   Gly Val Trp Asn Arg Lys Ala Asn Arg Lys Val Phe Gly Gln Trp Val
                   485                 490                 495
   Ser Ser Ser Thr Leu Asn Met Ser Glu Ala Ile Lys Gly Phe Leu Val
               500                 505                 510
   Lys Cys Cys Ala Tyr Asn Ser Leu Gly Thr Ser Cys Glu Thr Ile Leu
           515                 520                 525
   Leu Asn Ser Pro Gly Pro Phe Pro Phe Ile Gln Asp Asn Ile Ser Phe
       530                 535                 540
   Tyr Ala Thr Ile Gly Val Cys Leu Leu Phe Ile Val Val Leu Thr Leu
   545                 550                 555                 560
   Leu Ile Cys His Lys Tyr Lys Lys Gln Phe Arg Tyr Glu Ser Gln Leu
                   565                 570                 575
   Gln Met Val Gln Val Thr Gly Ser Ser Asp Asn Glu Tyr Phe Tyr Val
               580                 585                 590
   Asp Phe Arg Gly Ser Ser Asp Asn Glu Tyr Phe Tyr Val Asp Phe Arg
           595                 600                 605
   Glu Tyr Glu Tyr Asp Leu Lys Trp Glu Phe Pro Arg Glu Asn Leu Glu
       610                 615                 620
   Phe Gly Lys Val Leu Gly Ser Gly Ala Phe Gly Lys Val Met Asn Ala
   625                 630                 635                 640
   Thr Ala Tyr Gly Ile Ser Lys Thr Gly Val Ser Ile Gln Val Ala Val
                   645                 650                 655
   Lys Met Leu Lys Glu Lys Ala Asp Ser Ser Glu Arg Glu Ala Leu Met
               660                 665                 670
   Ser Glu Leu Lys Met Met Thr Gln Leu Gly Ser His Glu Asn Ile Val
           675                 680                 685
   Asn Leu Leu Gly Ala Cys Thr Leu Ser Gly Pro Ile Tyr Leu Ile Phe
       690                 695                 700
   Glu Tyr Cys Cys Tyr Gly Asp Leu Leu Asn Tyr Leu Arg Ser Lys Arg
   705                 710                 715                 720
   Glu Lys Phe His Arg Thr Trp Thr Glu Ile Phe Lys Glu His Asn Phe
                   725                 730                 735
   Ser Phe Tyr Pro Thr Phe Gln Ser His Pro Asn Ser Ser Met Pro Gly
               740                 745                 750
   Ser Arg Glu Val Gln Ile His Pro Asp Ser Asp Gln Ile Ser Gly Leu
           755                 760                 765
   His Gly Asn Ser Phe His Ser Glu Asp Glu Ile Glu Tyr Glu Asn Gln
       770                 775                 780
   Lys Arg Leu Glu Glu Glu Glu Asp Leu Asn Val Leu Thr Phe Glu Asp
   785                 790                 795                 800
   Leu Leu Cys Phe Ala Tyr Gln Val Ala Lys Gly Met Glu Phe Leu Glu
                   805                 810                 815
   Phe Lys Ser Cys Val His Arg Asp Leu Ala Ala Arg Asn Val Leu Val
               820                 825                 830
   Thr His Gly Lys Val Val Lys Ile Cys Asp Phe Gly Leu Ala Arg Asp
           835                 840                 845
   Ile Met Ser Asp Ser Asn Tyr Val Val Arg Gly Asn Ala Arg Leu Pro
       850                 855                 860
   Val Lys Trp Met Ala Pro Glu Ser Leu Phe Glu Gly Ile Tyr Thr Ile
   865                 870                 875                 880
   Lys Ser Asp Val Trp Ser Tyr Gly Ile Leu Leu Trp Glu Ile Phe Ser
                   885                 890                 895
   Leu Gly Val Asn Pro Tyr Pro Gly Ile Pro Val Asp Ala Asn Phe Tyr
               900                 905                 910
   Lys Leu Ile Gln Asn Gly Phe Lys Met Asp Gln Pro Phe Tyr Ala Thr
           915                 920                 925
   Glu Glu Ile Tyr Ile Ile Met Gln Ser Cys Trp Ala Phe Asp Ser Arg
       930                 935                 940
   Lys Arg Pro Ser Phe Pro Asn Leu Thr Ser Phe Leu Gly Cys Gln Leu
   945                 950                 955                 960
   Ala Asp Ala Glu Glu Ala Met Tyr Gln Asn Val Asp Gly Arg Val Ser
                   965                 970                 975
   Glu Cys Pro His Thr Tyr Gln Asn Arg Arg
               980                 985
(2) INFORMATION FOR SEQ ID NO: 21:
   (i) SEQUENCE CHARACTERISTICS:
      (A) LENGTH: 2978 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 21:
   ATGCCGGCGT TGGCGCGCGA CGCGGGCACC GTGCCGCTGC TCGTTGTTTT TTCTGCAATG      60
   ATATTTGGGA CTATTACAAA TCAAGATCTG CCTGTGATCA AGTGTGTTTT AATCAATCAT     120
   AAGAACAATG ATTCATCAGT GGGGAAGTCA TCATCATATC CCATGGTATC AGAATCCCCG     180
   GAAGACCTCG GGTGTGCGTT GAGACCCCAG AGCTCAGGGA CAGTGTACGA AGCTGCCGCT     240
   GTGGAAGTGG ATGTATCTGC TTCCATCACA CTGCAAGTGC TGGTCGATGC CCCAGGGAAC     300
   ATTTCCTGTC TCTGGGTCTT TAAGCACAGC TCCCTGAATT GCCAGCCACA TTTTGATTTA     360
   CAAAACAGAG GAGTTGTTTC CATGGTCATT TTGAAAATGA CAGAAACCCA AGCTGGAGAA     420
   TACCTACTTT TTATTCAGAG TGAAGCTACC AATTACACAA TATTGTTTAC AGTGAGTATA     480
   AGAAATACCC TGCTTTACAC ATTAAGAAGA CCTTACTTTA GAAAAATGGA AAACCAGGAC     540
   GCCCTGGTCT GCATATCTGA GAGCGTTCCA GAGCCGATCG TGGAATGGGT GCTTTGCGAT     600
   TCACAGGGGG AAAGCTGTAA AGAAGAAAGT CCAGCTGTTG TTAAAAAGGA GGAAAAAGTG     660
   CTTCATGAAT TATTTGGGAC GGACATAAGG TGCTGTGCCA GAAATGAACT GGGCAGGGAA     720
   TGCACCAGGC TGTTCACAAT AGATCTAAAT CAAACTCCTC AGACCACATT GCCACAATTA     780
   TTTCTTAAAG TAGGGGAACC CTTATGGATA AGGTGCAAAG CTGTTCATGT GAACCATGGA     840
   TTCGGGCTCA CCTGGGAATT AGAAAACAAA GCACTCGAGG AGGGCAACTA CTTTGAGATG     900
   AGTACCTATT CAACAAACAG AACTATGATA CGGATTCTGT TTGCTTTTGT ATCATCAGTG     960
   GCAAGAAACG ACACCGGATA CTACACTTGT TCCTCTTCAA AGCATCCCAG TCAATCAGCT    1020
   TTGGTTACCA TCGTAGGAAA GGGATTTATA AATGCTACCA ATTCAAGTGA AGATTATGAA    1080
   ATTGACCAAT ATGAAGAGTT TTGTTTTTCT GTCAGGTTTA AAGCCTACCC ACAAATCAGA    1140
   TGTACGTGGA CCTTCTCTCG AAAATCATTT CCTTGTGAGC AAAAGGGTCT TGATAACGGA    1200
   TACAGCATAT CCAAGTTTTG CAATCATAAG CACCAGCCAG GAGAATATAT ATTCCATGCA    1260
   GAAAATGATG ATGCCCAATT TACCAAAATG TTCACGCTGA ATATAAGAAG GAAACCTCAA    1320
   GTGCTCGCAG AAGCATCGGC AAGTCAGGCG TCCTGTTTCT CGGATGGATA CCCATTACCA    1380
   TCTTGGACCT GGAAGAAGTG TTCAGACAAG TCTCCCAACT GCACAGAAGA GATCACAGAA    1440
   GGAGTCTGGA ATAGAAAGGC TAACAGAAAA GTGTTTGGAC AGTGGGTGTC GAGCAGTACT    1500
   CTAAACATGA GTGAAGCCAT AAAAGGGTTC CTGGTCAAGT GCTGTGCATA CAATTCCCTT    1560
   GGCACATCTT GTGAGACGAT CCTTTTAAAC TCTCCAGGCC CCTTCCCTTT CATCCAAGAC    1620
   AACATCTCAT TCTATGCAAC AATTGGTGTT TGTCTCCTCT TCATTGTCGT TTTAACCCTG    1680
   CTAATTTGTC ACAAGTACAA AAAGCAATTT AGGTATGAAA GCCAGCTACA GATGGTACAG 1740
   GTGACCGGCT CCTCAGATAA TGAGTACTTC TACGTTGAAA GCCAGCTACA GATGGTACAG 1800
   GTGACCGGCT CCTCAGATAG TGAGTACTTC TACGTTGATT TCAGAGAATA TGAATATGAT 1860
   CTCAAATGGG AGTTTCCAAG AGAAAATTTA GAGTTTGGGA AGGTACTAGG ATCAGGTGCT 1920
   TTTGGAAAAG TGATGAACGC AACAGCTTTG GAATTAGCAA AACAGGAGTC TCAATCCAGG 1980
   TTGCCGTCAA AATGCTGAAA GAAAAAGCAG ACAGCTCTGA AAGAGAGGCA CTCATGTCAG 2040
   AACTCAAGAT GATGACCCAG CTGGGAAGCC ACGAGAATAT TGTGAACCTG CTGGGGGCGT 2100
   GCACACTGTC AGGACCAATT TACTTGATTT TTGAATACTG TTGCTATGGT GATCTTCTCA 2160
   ACTATCTAAG AAGTAAAAGA GAAAAATTTC ACAGGACTTG GACAGAGATT TTCAAGGAAC 2220
   ACAATTTCAG TTTTTACCCC ACTTTCCAAT CACATCCAAA TTCCAGCATG CCTGGTTCAA 2280
   GAGAAGTTCA GATACACCCG GACTCGGATC AAATCTCAGG GCTTCATGGG AATTCATTTC 2340
   ACTCTGAAGA TGAAATTGAA TATGAAAACC AAAAAAGGCT GGAAGAAGAG GAGGACTTGA 2400
   ATGTGCTTAC ATTTGAAGAT CTTCTTTGCT TTGCATATCA AGTTGCCAAA GGAATGGAAT 2460
   TTCTGGAATT TAAGTCGTGT GTTCACAGAG ACCTGGCCGC CAGGAACGTG CTTGTCACCC 2520
   ACGGGAAAGT GGTGAAGATA TGTGACTTTG GATTGGCTCG AGATATCATG AGTGATTCCA 2580
   ACTATGTTGT CAGGGGCAAT GCCCGTCTGC CTGTAAAATG GATGGCCCCC GAAAGCCTGT 2640
   TTGAAGGCAT CTACACCATT AAGAGTGATG TCTGGTCATA TGGAATATTA CTGTGGGAAA 2700
   TCTTCTCACT TGGTGTGAAT CCTTACCCTG GCATTCCGGT TGATGCTAAC TTCTACAAAC 2760
   TGATTCAAAA TGGATTTAAA ATGGATCAGC CATTTTATGC TACAGAAGAA ATATACATTA 2820
   TAATGCAATC CTGCTGGGCT TTTGACTCAA GGAAACGGCC ATCCTTCCCT AATTTGACTT 2880
   CGTTTTTAGG ATGTCAGCTG GCAGATGCAG AAGAAGCGAT GTATCAGAAT GTGGATGGCC 2940
   GTGTTTCGGA ATGTCCTCAC ACCTACCAAA ACAGGCGA 2978
(2) INFORMATION FOR SEQ ID NO: 22:
   (i) SEQUENCE CHARACTERISTICS:
      (A) LENGTH: 2982 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 22:
   ATGCCGGCGT TGGCGCGCGA CGCGGGCACC GTGCCGCTGC TCGTTGTTTT TTCTGCAATG      60
   ATATTTGGGA CTATTACAAA TCAAGATCTG CCTGTGATCA AGTGTGTTTT AATCAATCAT     120
   AAGAACAATG ATTCATCAGT GGGGAAGTCA TCATCATATC CCATGGTATC AGAATCCCCG     180
   GAAGACCTCG GGTGTGCGTT GAGACCCCAG AGCTCAGGGA CAGTGTACGA AGCTGCCGCT     240
   GTGGAAGTGG ATGTATCTGC TTCCATCACA CTGCAAGTGC TGGTCGATGC CCCAGGGAAC     300
   ATTTCCTGTC TCTGGGTCTT TAAGCACAGC TCCCTGAATT GCCAGCCACA TTTTGATTTA     360
   CAAAACAGAG GAGTTGTTTC CATGGTCATT TTGAAAATGA CAGAAACCCA AGCTGGAGAA     420
   TACCTACTTT TTATTCAGAG TGAAGCTACC AATTACACAA TATTGTTTAC AGTGAGTATA     480
   AGAAATACCC TGCTTTACAC ATTAAGAAGA CCTTACTTTA GAAAAATGGA AAACCAGGAC     540
   GCCCTGGTCT GCATATCTGA GAGCGTTCCA GAGCCGATCG TGGAATGGGT GCTTTGCGAT     600
   TCACAGGGGG AAAGCTGTAA AGAAGAAAGT CCAGCTGTTG TTAAAAAGGA GGAAAAAGTG     660
   CTTCATGAAT TATTTGGGAC GGACATAAGG TGCTGTGCCA GAAATGAACT GGGCAGGGAA     720
   TGCACCAGGC TGTTCACAAT AGATCTAAAT CAAACTCCTC AGACCACATT GCCACAATTA     780
   TTTCTTAAAG TAGGGGAACC CTTATGGATA AGGTGCAAAG CTGTTCATGT GAACCATGGA     840
   TTCGGGCTCA CCTGGGAATT AGAAAACAAA GCACTCGAGG AGGGCAACTA CTTTGAGATG     900
   AGTACCTATT CAACAAACAG AACTATGATA CGGATTCTGT TTGCTTTTGT ATCATCAGTG     960
   GCAAGAAACG ACACCGGATA CTACACTTGT TCCTCTTCAA AGCATCCCAG TCAATCAGCT    1020
   TTGGTTACCA TCGTAGGAAA GGGATTTATA AATGCTACCA ATTCAAGTGA AGATTATGAA    1080
   ATTGACCAAT ATGAAGAGTT TTGTTTTTCT GTCAGGTTTA AAGCCTACCC ACAAATCAGA    1140
   TGTACGTGGA CCTTCTCTCG AAAATCATTT CCTTGTGAGC AAAAGGGTCT TGATAACGGA    1200
   TACAGCATAT CCAAGTTTTG CAATCATAAG CACCAGCCAG GAGAATATAT ATTCCATGCA    1260
   GAAAATGATG ATGCCCAATT TACCAAAATG TTCACGCTGA ATATAAGAAG GAAACCTCAA    1320
   GTGCTCGCAG AAGCATCGGC AAGTCAGGCG TCCTGTTTCT CGGATGGATA CCCATTACCA    1380
   TCTTGGACCT GGAAGAAGTG TTCAGACAAG TCTCCCAACT GCACAGAAGA GATCACAGAA    1440
   GGAGTCTGGA ATAGAAAGGC TAACAGAAAA GTGTTTGGAC AGTGGGTGTC GAGCAGTACT    1500
   CTAAACATGA GTGAAGCCAT AAAAGGGTTC CTGGTCAAGT GCTGTGCATA CAATTCCCTT    1560
   GGCACATCTT GTGAGACGAT CCTTTTAAAC TCTCCAGGCC CCTTCCCTTT CATCCAAGAC    1620
   AACATCTCAT TCTATGCAAC AATTGGTGTT TGTCTCCTCT TCATTGTCGT TTTAACCCTG    1680
   CTAATTTGTC ACAAGTACAA AAAGCAATTT AGGTATGAAA GCCAGCTACA GATGGTACAG 1740
   GTGACCGGCT CCTCAGATAA TGAGTACTTC TACGTTGATT TCAGAGAATA TGAATATGAT 1800
   CTCAAATGGG AGTTTCCAAG AGAAAATTTA GAGTTTGGGA AGGTACTAGG ATCCGAATAT 1860
   GATCTCAAAT GGGAGTTTCC AAGAGAAAAT TTAGAGTTTG GGAAGGTACT AGGATCAGGT 1920
   GCTTTTGGAA AAGTGATGAA CGCAACAGCT TATGGAATTA GCAAAACAGG AGTCTCAATC 1980
   CAGGTTGCCG TCAAAATGCT GAAAGAAAAA GCAGACAGCT CTGAAAGAGA GGCACTCATG 2040
   TCAGAACTCA AGATGATGAC CCAGCTGGGA AGCCACGAGA ATATTGTGAA CCTGCTGGGG 2100
   GCGTGCACAC TGTCAGGACC AATTTACTTG ATTTTTGAAT ACTGTTGCTA TGGTGATCTT 2160
   CTCAACTATC TAAGAAGTAA AAGAGAAAAA TTTCACAGGA CTTGGACAGA GATTTTCAAG 2220
   GAACACAATT TCAGTTTTTA CCCCACTTTC CAATCACATC CAAATTCCAG CATGCCTGGT 2280
   TCAAGAGAAG TTCAGATACA CCCGGACTCG GATCAAATCT CAGGGCTTCA TGGGAATTCA 2340
   TTTCACTCTG AAGATGAAAT TGAATATGAA AACCAAAAAA GGCTGGAAGA AGAGGAGGAC 2400
   TTGAATGTGC TTACATTTGA AGATCTTCTT TGCTTTGCAT ATCAAGTTGC CAAAGGAATG 2460
   GAATTTCTGG AATTTAAGTC GTGTGTTCAC AGAGACCTGG CCGCCAGGAA CGTGCTTGTC 2520
   ACCCACGGGA AAGTGGTGAA GATATGTGAC TTTGGATTGG CTCGAGATAT CATGAGTGAT 2580
   TCCAACTATG TTGTCAGGGG CAATGCCCGT CTGCCTGTAA AATGGATGGC CCCCGAAAGC 2640
   CTGTTTGAAG GCATCTACAC CATTAAGAGT GATGTCTGGT CATATGGAAT ATTACTGTGG 2700
   GAAATCTTCT CACTTGGTGT GAATCCTTAC CCTGGCATTC CGGTTGATGC TAACTTCTAC 2760
   AAACTGATTC AAAATGGATT TAAAATGGAT CAGCCATTTT ATGCTACAGA AGAAATATAC 2820
   ATTATAATGC AATCCTGCTG GGCTTTTGAC TCAAGGAAAC GGCCATCCTT CCCTAATTTG 2880
   ACTTCGTTTT TAGGATGTCA GCTGGCAGAT GCAGAAGAAG CGATGTATCA GAATGTGGAT 2940
   GGCCGTGTTT CGGAATGTCC TCACACCTAC CAAAACAGGC GA 2982
(2) INFORMATION FOR SEQ ID NO: 23:
   (i) SEQUENCE CHARACTERISTICS:
      (A) LENGTH: 2958 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 23:
   ATGCCGGCGT TGGCGCGCGA CGCGGGCACC GTGCCGCTGC TCGTTGTTTT TTCTGCAATG 60
   ATATTTGGGA CTATTACAAA TCAAGATCTG CCTGTGATCA AGTGTGTTTT AATCAATCAT 120
   AAGAACAATG ATTCATCAGT GGGGAAGTCA TCATCATATC CCATGGTATC AGAATCCCCG 180
   GAAGACCTCG GGTGTGCGTT GAGACCCCAG AGCTCAGGGA CAGTGTACGA AGCTGCCGCT 240
   GTGGAAGTGG ATGTATCTGC TTCCATCACA CTGCAAGTGC TGGTCGATGC CCCAGGGAAC 300
   ATTTCCTGTC TCTGGGTCTT TAAGCACAGC TCCCTGAATT GCCAGCCACA TTTTGATTTA 360
   CAAAACAGAG GAGTTGTTTC CATGGTCATT TTGAAAATGA CAGAAACCCA AGCTGGAGAA 420
   TACCTACTTT TTATTCAGAG TGAAGCTACC AATTACACAA TATTGTTTAC AGTGAGTATA 480
   AGAAATACCC TGCTTTACAC ATTAAGAAGA CCTTACTTTA GAAAAATGGA AAACCAGGAC 540
   GCCCTGGTCT GCATATCTGA GAGCGTTCCA GAGCCGATCG TGGAATGGGT GCTTTGCGAT 600
   TCACAGGGGG AAAGCTGTAA AGAAGAAAGT CCAGCTGTTG TTAAAAAGGA GGAAAAAGTG 660
   CTTCATGAAT TATTTGGGAC GGACATAAGG TGCTGTGCCA GAAATGAACT GGGCAGGGAA 720
   TGCACCAGGC TGTTCACAAT AGATCTAAAT CAAACTCCTC AGACCACATT GCCACAATTA 780
   TTTCTTAAAG TAGGGGAACC CTTATGGATA AGGTGCAAAG CTGTTCATGT GAACCATGGA 840
   TTCGGGCTCA CCTGGGAATT AGAAAACAAA GCACTCGAGG AGGGCAACTA CTTTGAGATG 900
   AGTACCTATT CAACAAACAG AACTATGATA CGGATTCTGT TTGCTTTTGT ATCATCAGTG 960
   GCAAGAAACG ACACCGGATA CTACACTTGT TCCTCTTCAA AGCATCCCAG TCAATCAGCT 1020
   TTGGTTACCA TCGTAGGAAA GGGATTTATA AATGCTACCA ATTCAAGTGA AGATTATGAA 1080
   ATTGACCAAT ATGAAGAGTT TTGTTTTTCT GTCAGGTTTA AAGCCTACCC ACAAATCAGA 1140
   TGTACGTGGA CCTTCTCTCG AAAATCATTT CCTTGTGAGC AAAAGGGTCT TGATAACGGA 1200
   TACAGCATAT CCAAGTTTTG CAATCATAAG CACCAGCCAG GAGAATATAT ATTCCATGCA 1260
   GAAAATGATG ATGCCCAATT TACCAAAATG TTCACGCTGA ATATAAGAAG GAAACCTCAA 1320
   GTGCTCGCAG AAGCATCGGC AAGTCAGGCG TCCTGTTTCT CGGATGGATA CCCATTACCA 1380
   TCTTGGACCT GGAAGAAGTG TTCAGACAAG TCTCCCAACT GCACAGAAGA GATCACAGAA 1440
   GGAGTCTGGA ATAGAAAGGC TAACAGAAAA GTGTTTGGAC AGTGGGTGTC GAGCAGTACT 1500
   CTAAACATGA GTGAAGCCAT AAAAGGGTTC CTGGTCAAGT GCTGTGCATA CAATTCCCTT 1560
   GGCACATCTT GTGAGACGAT CCTTTTAAAC TCTCCAGGCC CCTTCCCTTT CATCCAAGAC 1620
   AACATCTCAT TCTATGCAAC AATTGGTGTT TGTCTCCTCT TCATTGTCGT TTTAACCCTG 1680
   CTAATTTGTC ACAAGTACAA AAAGCAATTT AGGTATGAAA GCCAGCTACA GATGGTACAG    1740
   GTGACCGGCT CCTCAGATAA TGAGTACTTC TACGTTGATT TCAGAGAATA TGAATATGAT    1800
   CTCAAATGGG AGTTTGATTT CAGAGAATAT GAATATGATC TCAAATGGGA GTTTCCAAGA    1860
   GAAAATTTAG AGTTTGGGAA GGTACTAGGA TCAGGTGCTT TTGGAAAAGT GATGAACGCA    1920
   ACAGCTTATG GAATTAGCAA AACAGGAGTC TCAATCCAGG TTGCCGTCAA AATGCTGAAA    1980
   GAAAAAGCAG ACAGCTCTGA AAGAGAGGCA CTCATGTCAG AACTCAAGAT GATGACCCAG    2040
   CTGGGAAGCC ACGAGAATAT TGTGAACCTG CTGGGGGCGT GCACACTGTC AGGACCAATT    2100
   TACTTGATTT TTGAATACTG TTGCTATGGT GATCTTCTCA ACTATCTAAG AAGTAAAAGA    2160
   GAAAAATTTC ACAGGACTTG GACAGAGATT TTCAAGGAAC ACAATTTCAG TTTTTACCCC    2220
   ACTTTCCAAT CACATCCAAA TTCCAGCATG CCTGGTTCAA GAGAAGTTCA GATACACCCG    2280
   GACTCGGATC AAATCTCAGG GCTTCATGGG AATTCATTTC ACTCTGAAGA TGAAATTGAA    2340
   TATGAAAACC AAAAAAGGCT GGAAGAAGAG GAGGACTTGA ATGTGCTTAC ATTTGAAGAT    2400
   CTTCTTTGCT TTGCATATCA AGTTGCCAAA GGAATGGAAT TTCTGGAATT TAAGTCGTGT    2460
   GTTCACAGAG ACCTGGCCGC CAGGAACGTG CTTGTCACCC ACGGGAAAGT GGTGAAGATA    2520
   TGTGACTTTG GATTGGCTCG AGATATCATG AGTGATTCCA ACTATGTTGT CAGGGGCAAT    2580
   GCCCGTCTGC CTGTAAAATG GATGGCCCCC GAAAGCCTGT TTGAAGGCAT CTACACCATT    2640
   AAGAGTGATG TCTGGTCATA TGGAATATTA CTGTGGGAAA TCTTCTCACT TGGTGTGAAT    2700
   CCTTACCCTG GCATTCCGGT TGATGCTAAC TTCTACAAAC TGATTCAAAA TGGATTTAAA    2760
   ATGGATCAGC CATTTTATGC TACAGAAGAA ATATACATTA TAATGCAATC CTGCTGGGCT    2820
   TTTGACTCAA GGAAACGGCC ATCCTTCCCT AATTTGACTT CGTTTTTAGG ATGTCAGCTG    2880
   GCAGATGCAG AAGAAGCGAT GTATCAGAAT GTGGATGGCC GTGTTTCGGA ATGTCCTCAC    2940
   ACCTACCAAA ACAGGCGA                                                  2958
(2) INFORMATION FOR SEQ ID NO: 24:
   (i) SEQUENCE CHARACTERISTICS:
      (A) LENGTH: 2949 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 24:
   ATGCCGGCGT TGGCGCGCGA CGCGGGCACC GTGCCGCTGC TCGTTGTTTT TTCTGCAATG 60
   ATATTTGGGA CTATTACAAA TCAAGATCTG CCTGTGATCA AGTGTGTTTT AATCAATCAT 120
   AAGAACAATG ATTCATCAGT GGGGAAGTCA TCATCATATC CCATGGTATC AGAATCCCCG 180
   GAAGACCTCG GGTGTGCGTT GAGACCCCAG AGCTCAGGGA CAGTGTACGA AGCTGCCGCT 240
   GTGGAAGTGG ATGTATCTGC TTCCATCACA CTGCAAGTGC TGGTCGATGC CCCAGGGAAC 300
   ATTTCCTGTC TCTGGGTCTT TAAGCACAGC TCCCTGAATT GCCAGCCACA TTTTGATTTA 360
   CAAAACAGAG GAGTTGTTTC CATGGTCATT TTGAAAATGA CAGAAACCCA AGCTGGAGAA 420
   TACCTACTTT TTATTCAGAG TGAAGCTACC AATTACACAA TATTGTTTAC AGTGAGTATA 480
   AGAAATACCC TGCTTTACAC ATTAAGAAGA CCTTACTTTA GAAAAATGGA AAACCAGGAC 540
   GCCCTGGTCT GCATATCTGA GAGCGTTCCA GAGCCGATCG TGGAATGGGT GCTTTGCGAT 600
   TCACAGGGGG AAAGCTGTAA AGAAGAAAGT CCAGCTGTTG TTAAAAAGGA GGAAAAAGTG 660
   CTTCATGAAT TATTTGGGAC GGACATAAGG TGCTGTGCCA GAAATGAACT GGGCAGGGAA 720
   TGCACCAGGC TGTTCACAAT AGATCTAAAT CAAACTCCTC AGACCACATT GCCACAATTA 780
   TTTCTTAAAG TAGGGGAACC CTTATGGATA AGGTGCAAAG CTGTTCATGT GAACCATGGA 840
   TTCGGGCTCA CCTGGGAATT AGAAAACAAA GCACTCGAGG AGGGCAACTA CTTTGAGATG 900
   AGTACCTATT CAACAAACAG AACTATGATA CGGATTCTGT TTGCTTTTGT ATCATCAGTG 960
   GCAAGAAACG ACACCGGATA CTACACTTGT TCCTCTTCAA AGCATCCCAG TCAATCAGCT 1020
   TTGGTTACCA TCGTAGGAAA GGGATTTATA AATGCTACCA ATTCAAGTGA AGATTATGAA 1080
   ATTGACCAAT ATGAAGAGTT TTGTTTTTCT GTCAGGTTTA AAGCCTACCC ACAAATCAGA 1140
   TGTACGTGGA CCTTCTCTCG AAAATCATTT CCTTGTGAGC AAAAGGGTCT TGATAACGGA 1200
   TACAGCATAT CCAAGTTTTG CAATCATAAG CACCAGCCAG GAGAATATAT ATTCCATGCA 1260
   GAAAATGATG ATGCCCAATT TACCAAAATG TTCACGCTGA ATATAAGAAG GAAACCTCAA 1320
   GTGCTCGCAG AAGCATCGGC AAGTCAGGCG TCCTGTTTCT CGGATGGATA CCCATTACCA 1380
   TCTTGGACCT GGAAGAAGTG TTCAGACAAG TCTCCCAACT GCACAGAAGA GATCACAGAA 1440
   GGAGTCTGGA ATAGAAAGGC TAACAGAAAA GTGTTTGGAC AGTGGGTGTC GAGCAGTACT 1500
   CTAAACATGA GTGAAGCCAT AAAAGGGTTC CTGGTCAAGT GCTGTGCATA CAATTCCCTT 1560
   GGCACATCTT GTGAGACGAT CCTTTTAAAC TCTCCAGGCC CCTTCCCTTT CATCCAAGAC 1620
   AACATCTCAT TCTATGCAAC AATTGGTGTT TGTCTCCTCT TCATTGTCGT TTTAACCCTG 1680
   CTAATTTGTC ACAAGTACAA AAAGCAATTT AGGTATGAAA GCCAGCTACA GATGGTACAG    1740
   GTGACCGGCT CCTCAGATAA TGAGTACTTC TACGTTGATT TCAGAGAATA TGAATATGAT    1800
   CTCAAATGGG AGTTTCCAAG AGAAAATTGG CACAAATGGG AGTTTCCAAG AGAAAATTTA    1860
   GAGTTTGGGA AGGTACTAGG ATCAGGTGCT TTTGGAAAAG TGATGAACGC AACAGCTTAT    1920
   GGAATTAGCA AAACAGGAGT CTCAATCCAG GTTGCCGTCA AAATGCTGAA AGAAAAAGCA    1980
   GACAGCTCTG AAAGAGAGGC ACTCATGTCA GAACTCAAGA TGATGACCCA GCTGGGAAGC    2040
   CACGAGAATA TTGTGAACCT GCTGGGGGCG TGCACACTGT CAGGACCAAT TTACTTGATT    2100
   TTTGAATACT GTTGCTATGG TGATCTTCTC AACTATCTAA GAAGTAAAAG AGAAAAATTT    2160
   CACAGGACTT GGACAGAGAT TTTCAAGGAA CACAATTTCA GTTTTTACCC CACTTTCCAA    2220
   TCACATCCAA ATTCCAGCAT GCCTGGTTCA AGAGAAGTTC AGATACACCC GGACTCGGAT    2280
   CAAATCTCAG GGCTTCATGG GAATTCATTT CACTCTGAAG ATGAAATTGA ATATGAAAAC    2340
   CAAAAAAGGC TGGAAGAAGA GGAGGACTTG AATGTGCTTA CATTTGAAGA TCTTCTTTGC    2400
   TTTGCATATC AAGTTGCCAA AGGAATGGAA TTTCTGGAAT TTAAGTCGTG TGTTCACAGA    2460
   GACCTGGCCG CCAGGAACGT GCTTGTCACC CACGGGAAAG TGGTGAAGAT ATGTGACTTT    2520
   GGATTGGCTC GAGATATCAT GAGTGATTCC AACTATGTTG TCAGGGGCAA TGCCCGTCTG    2580
   CCTGTAAAAT GGATGGCCCC CGAAAGCCTG TTTGAAGGCA TCTACACCAT TAAGAGTGAT    2640
   GTCTGGTCAT ATGGAATATT ACTGTGGGAA ATCTTCTCAC TTGGTGTGAA TCCTTACCCT    2700
   GGCATTCCGG TTGATGCTAA CTTCTACAAA CTGATTCAAA ATGGATTTAA AATGGATCAG    2760
   CCATTTTATG CTACAGAAGA AATATACATT ATAATGCAAT CCTGCTGGGC TTTTGACTCA    2820
   AGGAAACGGC CATCCTTCCC TAATTTGACT TCGTTTTTAG GATGTCAGCT GGCAGATGCA    2880
   GAAGAAGCGA TGTATCAGAA TGTGGATGGC CGTGTTTCGG AATGTCCTCA CACCTACCAA    2940
   AACAGGCGA                                                            2949
(2) INFORMATION FOR SEQ ID NO: 25:
   (i) SEQUENCE CHARACTERISTICS:
      (A) LENGTH: 2958 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (xi) SEQUENCE DESCRIPTION: SEQ ID NO: 25:
   ATGCCGGCGT TGGCGCGCGA CGCGGGCACC GTGCCGCTGC TCGTTGTTTT TTCTGCAATG      60
   ATATTTGGGA CTATTACAAA TCAAGATCTG CCTGTGATCA AGTGTGTTTT AATCAATCAT     120
   AAGAACAATG ATTCATCAGT GGGGAAGTCA TCATCATATC CCATGGTATC AGAATCCCCG     180
   GAAGACCTCG GGTGTGCGTT GAGACCCCAG AGCTCAGGGA CAGTGTACGA AGCTGCCGCT     240
   GTGGAAGTGG ATGTATCTGC TTCCATCACA CTGCAAGTGC TGGTCGATGC CCCAGGGAAC     300
   ATTTCCTGTC TCTGGGTCTT TAAGCACAGC TCCCTGAATT GCCAGCCACA TTTTGATTTA     360
   CAAAACAGAG GAGTTGTTTC CATGGTCATT TTGAAAATGA CAGAAACCCA AGCTGGAGAA     420
   TACCTACTTT TTATTCAGAG TGAAGCTACC AATTACACAA TATTGTTTAC AGTGAGTATA     480
   AGAAATACCC TGCTTTACAC ATTAAGAAGA CCTTACTTTA GAAAAATGGA AAACCAGGAC     540
   GCCCTGGTCT GCATATCTGA GAGCGTTCCA GAGCCGATCG TGGAATGGGT GCTTTGCGAT     600
   TCACAGGGGG AAAGCTGTAA AGAAGAAAGT CCAGCTGTTG TTAAAAAGGA GGAAAAAGTG     660
   CTTCATGAAT TATTTGGGAC GGACATAAGG TGCTGTGCCA GAAATGAACT GGGCAGGGAA     720
   TGCACCAGGC TGTTCACAAT AGATCTAAAT CAAACTCCTC AGACCACATT GCCACAATTA     780
   TTTCTTAAAG TAGGGGAACC CTTATGGATA AGGTGCAAAG CTGTTCATGT GAACCATGGA     840
   TTCGGGCTCA CCTGGGAATT AGAAAACAAA GCACTCGAGG AGGGCAACTA CTTTGAGATG     900
   AGTACCTATT CAACAAACAG AACTATGATA CGGATTCTGT TTGCTTTTGT ATCATCAGTG     960
   GCAAGAAACG ACACCGGATA CTACACTTGT TCCTCTTCAA AGCATCCCAG TCAATCAGCT    1020
   TTGGTTACCA TCGTAGGAAA GGGATTTATA AATGCTACCA ATTCAAGTGA AGATTATGAA    1080
   ATTGACCAAT ATGAAGAGTT TTGTTTTTCT GTCAGGTTTA AAGCCTACCC ACAAATCAGA    1140
   TGTACGTGGA CCTTCTCTCG AAAATCATTT CCTTGTGAGC AAAAGGGTCT TGATAACGGA    1200
   TACAGCATAT CCAAGTTTTG CAATCATAAG CACCAGCCAG GAGAATATAT ATTCCATGCA    1260
   GAAAATGATG ATGCCCAATT TACCAAAATG TTCACGCTGA ATATAAGAAG GAAACCTCAA    1320
   GTGCTCGCAG AAGCATCGGC AAGTCAGGCG TCCTGTTTCT CGGATGGATA CCCATTACCA    1380
   TCTTGGACCT GGAAGAAGTG TTCAGACAAG TCTCCCAACT GCACAGAAGA GATCACAGAA    1440
   GGAGTCTGGA ATAGAAAGGC TAACAGAAAA GTGTTTGGAC AGTGGGTGTC GAGCAGTACT    1500
   CTAAACATGA GTGAAGCCAT AAAAGGGTTC CTGGTCAAGT GCTGTGCATA CAATTCCCTT    1560
   GGCACATCTT GTGAGACGAT CCTTTTAAAC TCTCCAGGCC CCTTCCCTTT CATCCAAGAC    1620
   AACATCTCAT TCTATGCAAC AATTGGTGTT TGTCTCCTCT TCATTGTCGT TTTAACCCTG    1680
   CTAATTTGTC ACAAGTACAA AAAGCAATTT AGGTATGAAA GCCAGCTACA GATGGTACAG 1740
   GTGACCGGCT CCTCAGATAA TGAGTACTTC TACGTTGATT TCAGAGGCTC CTCAGATAAT 1800
   GAGTACTTCT ACGTTGATTT CAGAGAATAT GAATATGATC TCAAATGGGA GTTTCCAAGA 1860
   GAAAATTTAG AGTTTGGGAA GGTACTAGGA TCAGGTGCTT TTGGAAAAGT GATGAACGCA 1920
   ACAGCTTATG GAATTAGCAA AACAGGAGTC TCAATCCAGG TTGCCGTCAA AATGCTGAAA 1980
   GAAAAAGCAG ACAGCTCTGA AAGAGAGGCA CTCATGTCAG AACTCAAGAT GATGACCCAG 2040
   CTGGGAAGCC ACGAGAATAT TGTGAACCTG CTGGGGGCGT GCACACTGTC AGGACCAATT 2100
   TACTTGATTT TTGAATACTG TTGCTATGGT GATCTTCTCA ACTATCTAAG AAGTAAAAGA 2160
   GAAAAATTTC ACAGGACTTG GACAGAGATT TTCAAGGAAC ACAATTTCAG TTTTTACCCC 2220
   ACTTTCCAAT CACATCCAAA TTCCAGCATG CCTGGTTCAA GAGAAGTTCA GATACACCCG 2280
   GACTCGGATC AAATCTCAGG GCTTCATGGG AATTCATTTC ACTCTGAAGA TGAAATTGAA 2340
   TATGAAAACC AAAAAAGGCT GGAAGAAGAG GAGGACTTGA ATGTGCTTAC ATTTGAAGAT 2400
   CTTCTTTGCT TTGCATATCA AGTTGCCAAA GGAATGGAAT TTCTGGAATT TAAGTCGTGT 2460
   GTTCACAGAG ACCTGGCCGC CAGGAACGTG CTTGTCACCC ACGGGAAAGT GGTGAAGATA 2520
   TGTGACTTTG GATTGGCTCG AGATATCATG AGTGATTCCA ACTATGTTGT CAGGGGCAAT 2580
   GCCCGTCTGC CTGTAAAATG GATGGCCCCC GAAAGCCTGT TTGAAGGCAT CTACACCATT 2640
   AAGAGTGATG TCTGGTCATA TGGAATATTA CTGTGGGAAA TCTTCTCACT TGGTGTGAAT 2700
   CCTTACCCTG GCATTCCGGT TGATGCTAAC TTCTACAAAC TGATTCAAAA TGGATTTAAA 2760
   ATGGATCAGC CATTTTATGC TACAGAAGAA ATATACATTA TAATGCAATC CTGCTGGGCT 2820
   TTTGACTCAA GGAAACGGCC ATCCTTCCCT AATTTGACTT CGTTTTTAGG ATGTCAGCTG 2880
   GCAGATGCAG AAGAAGCGAT GTATCAGAAT GTGGATGGCC GTGTTTCGGA ATGTCCTCAC 2940
   ACCTACCAAA ACAGGCGA 2958
(2) INFORMATION FOR SEQ ID NO: 26:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 26:
      TGTCGAGCAG TACTCTAAAC A 21
(2) INFORMATION FOR SEQ ID NO: 27:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 27:
      ATCCTAGTAC CTTCCCAAAC TC 22
(2) INFORMATION FOR SEQ ID NO: 28:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 28:
      CTTCCTGGGC ATGGAGTC 18
(2) INFORMATION FOR SEQ ID NO: 29:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 29:
      CGCTCAGGAG GAGCAATGAT 20
(2) INFORMATION FOR SEQ ID NO: 30:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 30:
      CAATTTAGGT ATGAAAGCC 19
(2) INFORMATION FOR SEQ ID NO: 31:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 31:
      CAAACTCTAA ATTTTCTCT 19
(2) INFORMATION FOR SEQ ID NO: 32:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 32:
      TGTCTTTGCA GGGAAGGTTA C 21
(2) INFORMATION FOR SEQ ID NO: 33:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid (synthetic DNA)
   (iii) HYPOTHETICAL: NO
   (xi) SEQUENCE DESCRIPTION: SEQ ID NO: 33:
      GTACCTTTCA GCATTTTGAC 20